# FASCICULE DE BREVET EUROPEEN

(11) **EP 0 516 729 B1**
(45) Date de publication et mention de la délivrance du brevet: **29.12.1993**
(21) Numéro de dépôt: 91905298.5
(22) Date de dépôt: 14.02.1991
(51) Int. Cl.: C07K 5/06, A61K 37/02, C07C 275/28

(54) **N-PHENYL N-ALCOXYCARBONYLALKYLE GLYCINAMIDES, LEUR PREPARATION ET LES MEDICAMENTS LES CONTENANT**
N-PHENYL-N-ALKOXYCARBONYLALKYL-GLYCINAMIDE, IHRE HERSTELLUNG UND SIE ENTHALTENDE MEDIKAMENTE
N-PHENYL N-ALKOXYCARBONYLALKYL GLYCINAMIDES, PREPARATION METHOD AND DRUGS CONTAINING THEM

(30) Priorité: 19.02.1990 FR 9001961
(43) Date de publication de la demande: 09.12.1992
(73) Titulaire: RHONE-POULENC RORER S.A., 92165 Antony Cédex (FR)
(72) Inventeur: BOURZAT, Jean-Dominique, F-94300 Vincennes (FR); CAPET, Marc, F-94320 Thiais (FR); COTREL, Claude, F-75012 Paris (FR); GUYON, Claude, F-94100 S.-Maur-des-Fosses (FR); MANFRE, Franco, F-94400 Vitry-sur-Seine (FR); ROUSSEL, Gérard, F-91450 Soisy-sur-Seine (FR)
(74) Mandataire: Pilard, Jacques
(86) Numéro de dépôt international: FR9100119
(87) Numéro de publication internationale: WO9112265

(56) Documents cités:
- WO-A-91/12264
- WO-A-91/13907
- PEPTIDES, Proceedings of the 9th American Peptide Symposium, 1985, Pierce Chemical Co., Rockford, IL (US); D.E. PORTLOCK et al., pp. 128-133#
- CHEMICAL ABSTRACTS, vol. 105, 1986, Columbus, OH (US); p. 801, no. 173038h#
- CHEMICAL ABSTRACTS, vol. 113, 1990, Columbus, OH (US); T. LU et al., p. 784, no. 231990r#

## Description

La présente invention concerne des composés de formule: 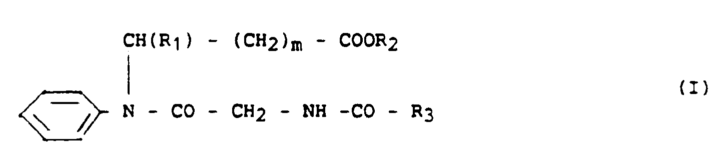
leur préparation et les médicaments les contenant.

Dans la formule (I),
- R₁ représente un atome d'hydrogène ou un radical alkyle, alcoxycarbonyle ou phényle non substitué ou substitué par un ou plusieurs substituants choisis parmi les atomes d'halogène et les radicaux alkyle, alcoxy, nitro, amino et alkylthio,
- R₂ représente un radical alkyle (1-8C), polyfluoroalkyle, cinnamyle ou cycloalkyle non substitué ou substitué par un ou plusieurs radicaux alkyle,
- R₃ représente un radical phényle (non substitué ou substitué par un ou plusieurs substituants choisis parmi les radicaux alkyle, alcoxy et alkylthio et les atomes d'halogène), un radical naphtyle, indolyle, quinolyle, phénylamino (dont le noyau phényle est éventuellement substitué par un ou plusieurs subtituants choisis parmi les atomes d'halogène et les radicaux alkyle, alcoxy et alkylthio) ou un radical quinolylamino,
- m est égal à 0 ou 1,

étant entendu que lorsque R₁ représente un atome d'hydrogène ou un radical alkyle ou phényle, R₃ représente un radical naphtyle, indolyle ou phénylamino éventuellement substitué par un radical alkyle, alcoxy, alkylthio ou par un ou deux atomes d'halogène et m est égal à 0, R₂ n'est pas un radical alkyle contenant 1 à 4 atomes de carbone ou un radical cycloalkyle.

Dans les définitions qui précèdent et celles qui seront citées ci-après, sauf mention contraire, les radicaux alkyle et alcoxy et les portions alkyle et alcoxy contiennent 1 à 4 atomes de carbone en chaîne droite ou ramifiée et les radicaux cycloalkyle contiennent 3 à 6 atomes de carbone.

Dans la formule (I), les atomes d'halogène sont de préférence les atomes de chlore, de brome ou de fluor.

Les composés de formule (I) contenant un ou plusieurs centres asymétriques présentent des formes isomères. Les racémiques et les énantiomères de ces composés font également partie de l'invention.

Les composés de formule (I) pour lesquels R₃ représente un radical phénylamino dont le noyau phényle est éventuellement substitué peuvent être préparés par action d'un acide de formule :

HOOC - CH₂ - NH - CO - R₃ (II)

dans laquelle R₃ a les mêmes significations que précédemment sur une amine de formule : 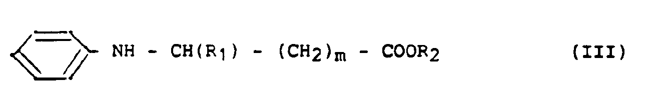
dans laquelle m, R₁ et R₂ ont les mêmes significations que dans la formule (I).

Cette réaction s'effectue généralement au sein d'un solvant inerte tel qu'un solvant chloré (chloroforme, dichlorométhane, dichloro-1,2 éthane par exemple), au moyen de chlorure de thionyle, à la température d'ébullition du solvant.

Les acides de formule (II) peuvent être obtenus par action d'un phénylisocyanate dont le noyau phényle est éventuellement substitué par ou plusieurs substituants choisis parmi les atomes d'halogène et les radicaux alkyle, alcoxy et alkylthio, sur un dérivé de formule :

HOOC - CH₂ - NH₂ (IV)

Cette réaction s'effectue généralement en milieu aqueux, en présence d'un agent alcalin tel qu'un bicarbonate de métal alcalin, à une température comprise entre 15 et 30°C.

Les amines de formule (III) pour lesquelles m est égal à 0 peuvent être obtenues par action d'aniline sur un dérivé de formule:

Hal - CH(R₁) - (CH₂)ₘ - COOR₂ (V)

dans laquelle R₁ a les mêmes significations que dans la formule (I), m est égal à 0 et Hal représente un atome d'halogène (de préférence chlore ou brome).

Cette réaction s'effectue de préférence au sein d'un solvant inerte tel que l'acétonitrile, le diméthylformamide ou le tétrahydrofuranne, à la température d'ébullition du solvant.

Les dérivés de formule (V), pour lesquels R₁ représente un radical alkyle, alcoxycarbonyle ou phényle substitué peuvent être obtenus par halogénation d'un dérivé de formule :

HCH(R₁) - (CH₂)ₘ - COOR₂ (VI)

dans laquelle R₁ a les mêmes significations que précédemment, R₂ a les mêmes significations que dans la formule (I) et m est égal à 0.

Cette halogénation s'effectue au moyen d'un agent d'halogénation tel que le N-bromosuccinimide, le N-chlorosuccinimide ou le brome en présence éventuellement d'azo-bis-isobutyronitrile, ou d'acétamide, au sein d'un solvant inerte tel qu'un solvant chloré (chloroforme, tétrachlorure de carbone par exemple), à une température comprise entre 20°C et la température d'ébullition du solvant.

Les dérivés de formule (VI) peuvent être obtenus par action d'un alcool de formule :

HOR₂ (VII)

dans laquelle R₂ a les mêmes significations que dans la formule (I), sur un acide de formule :

HCH(R₁) - (CH₂)ₘ - COOH (VIII)

dans laquelle R₁ a les mêmes significations que dans la formule (VI) et m est égal à 0 ou un dérivé réactif de cet acide tel qu'un halogénure.

Lorsque l'on met en oeuvre l'acide, on opère en présence d'un acide tel que l'acide sulfurique, à la température d'ébullition du milieu réactionnel.

Lorsque l'on met en oeuvre un halogénure, on opère en présence d'une amine tertiaire telle que la triéthylamine ou la N,N-diméthylaniline, à une température voisine de 20°C.

Les dérivés de formule (VIII) pour lesquels R₁ représente un radical alcoxycarbonyle peuvent être obtenus par application ou adaptation de la méthode décrite dans Acta Chem. Scand., B29, 687 (1975).

Les dérivés de formule (V) pour lesquels m est égal à 0 peuvent également être obtenus par action d'un alcool de formule (VII) sur un dérivé dihalogéné de formule :

Hal - CH(R₁) - (CH₂)ₘ - COHal (IX)

dans laquelle R₁ a les mêmes significations que dans la formule (I), m est égal à 0 et Hal représente un atome d'halogène.

Cette réaction s'effectue en présence d'une amine tertiaire telle que la triéthylamine ou la N,N-diméthylaniline, à une température comprise entre 0 et 30°C, au sein d'un solvant inerte tel qu'un solvant chloré (chloroforme, dichloro-1,2 éthane par exemple) ou d'un éther (éther diéthylique par exemple).

Les dérivés dihalogénés de formule (IX) peuvent être obtenus par halogénation d'un dérivé de formule :

HCH(R₁) - (CH₂)ₘ - COHal (X)

dans laquelle R₁ a les mêmes significations que dans la formule (I), m est égal à O et Hal représente un atome d'halogène.

Cette halogénation s'effectue généralement dans les conditions mentionnées précédemment pour l'halogénation des composés de formule (VI).

Les dérivés de formule (X) peuvent être obtenus par halogénation des acides correspondants par toute méthode connue de l'homme de l'art pour passer d'un acide à un halogénure d'acide. De préférence, on fait réagir le chlorure de thionyle ou le bromure de thionyle.

Les dérivés de formule (X) pour lesquels R₁ représente un radical alcoxycarbonyle peuvent également être obtenus par application ou adaptation de la méthode décrite dans Beilstein, 2, 582.

Les amines de formule (III) pour lesquelles m est égal à 1 peuvent être obtenues par réduction d'un dérivé de formule : 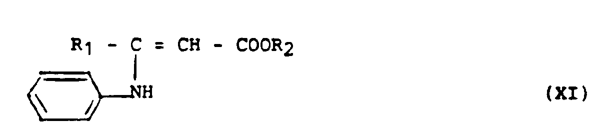
dans laquelle R₁ et R₂ ont les mêmes significations que dans la formule (I).

Cette réaction s'effectue généralement au sein d'un solvant inerte tel que le tétrahydrofuranne, au moyen d'acide trifluoroacétique et de cyanoborohydrure de sodium en solution dans le méthanol, à une température voisine de 0°C.

Les dérivés de formule (XI) peuvent être obtenus par application ou adaptation de la méthode décrite par F. TEXIER-BOULLET, Synthesis, 679 (1985).

Les composés de formule (I) pour lesquels R₃ représente un radical phénylamino dont le noyau phényle est éventuellement substitué ou quinolylamino peuvent également être préparés par action d'un dérivé aminé de formule : 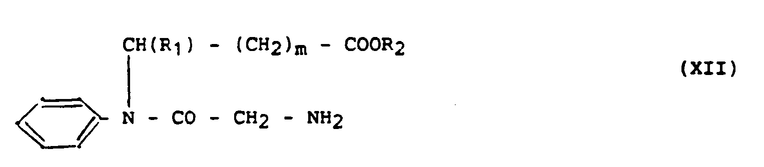
dans laquelle R₁, R₂ et m ont les mêmes significations que dans la formule (I) sur un un dérivé de formule :

R₄ - NH -COOCCl₃ (XIII)

dans laquelle R₄ représente un radical phényle (éventuellement substitué par un ou plusieurs substituants choisis parmi les atomes d'halogène et les radicaux alkyle, alcoxy et alkylthio) ou quinolyle.

Cette réaction s'effectue généralement au sein d'un solvant inerte tel qu'un solvant chloré (chloroforme, dichlorométhane par exemple), un solvant aromatique (benzène, toluène par exemple) à une température voisine de 20°C.

Les dérivés aminés de formule (XII) peuvent être obtenus par action d'hydrazine ou de méthylhydrazine sur un dérivé de formule : 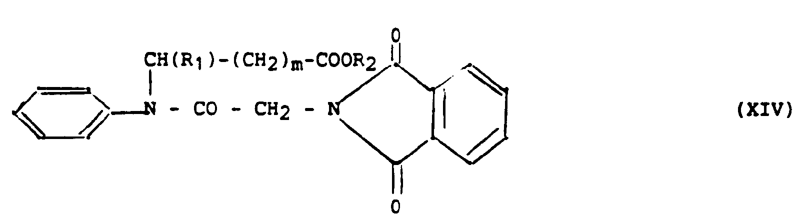
dans laquelle R₁, R₂ et m ont les mêmes significations que dans la formule (I).

Cette réaction s'effectue généralement au sein d'un solvant inerte tel qu'un alcool (méthanol, éthanol par exemple), un solvant chloré (dichlorométhane, chloroforme par exemple), à une température comprise entre 0°C et la température d'ébullition du solvant.

Les dérivés de formule (XIV) peuvent être obtenus par action d'un chlorure de formule : 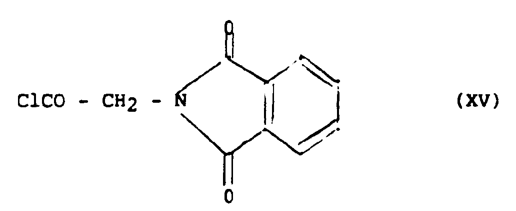
sur un dérivé de formule (III) dans laquelle R_{1,} R₂ et m ont les mêmes significations que dans la formule (I).

Cette réaction s'effectue généralement au sein d'un solvant inerte tel qu'un solvant chloré (chloroforme, dichloro-1,2 éthane par exemple), en présence d'un agent alcalin tel qu'un bicarbonate de métal alcalin, à une température comprise entre 20°C et la température d'ébullition du solvant.

Le chlorure de formule (XV) peut être obtenu par application de la méthode décrite par W. GRASSMANN et coll., Chem. Ber., 83, 244 (1950).

Les dérivés de formule (XIII). peuvent être obtenus par action du chloroformiate de trichlorométhyle sur une amine de formule :

R₄ - NH₂ (XVI)

dans laquelle R₄ a les mêmes significations que dans la formule (XIII).

Cette réaction s'effectue de préférence au sein d'un solvant inerte tel qu'un solvant chloré (chloroforme, dichlorométhane par exemple), en présence d'une base telle qu'une trialkylamine, à une température voisine de 0°C.

Les composés de formule (I) pour lesquels R₃ représente un radical phényle éventuellement substitué, naphtyle, indolyle ou quinolyle peuvent être préparés par action d'un dérivé aminé de formule (XII) sur un acide de formule :

HOOC - R₃ (XVII)

dans laquelle R₃ a les mêmes significations que précédemment ou un dérivé réactif de cet acide.

Lorsqu'on met en oeuvre l'acide, on opère en présence d'un agent de condensation peptidique tel qu'un carbodiimide (par exemple le dicyclohexylcarbodiimide ou le N,N'-carbonyldiimidazole), au sein d'un solvant inerte tel qu'un éther (par exemple tétrahydrofuranne, dioxanne), un amide (diméthylformamide par exemple) ou un solvant chloré (chloroforme, dichlorométhane par exemple) à une température comprise entre 0°C et la température d'ébullition du solvant.

Lorsqu'on met en oeuvre un dérivé de l'acide, il est possible de faire réagir l'anhydride, un anhydride mixte, un halogénure d'acide ou un ester (qui peut être choisi parmi les esters activés ou non de l'acide). On opère alors soit en milieu organique éventuellement en présence d'un accepteur d'acide tel qu'une base organique azotée (par exemple une trialkylamine, une pyridine, le diaza-1,8 bicyclo [5.4.0] undécène-7 ou le diaza-1,5 bicyclo [4.3.0] nonène-5), dans un solvant tel que cité ci-dessus ou un mélange de ces solvants, à une température comprise entre 0°C et le température de reflux du mélange réactionnel ; soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les composés de formule (I) peuvent également être préparés par estérification d'un acide de formule : 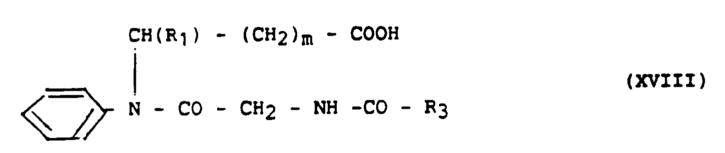
dans laquelle R₁, R₃ et m ont les mêmes significations que dans la formule (I) au moyen d'un alcool de formule (VII).

Cette estérification s'effectue généralement au sein d'un solvant inerte tel qu'un solvant chloré (chloroforme, chlorure de méthylène par exemple), en présence d'une base telle qu'une trialkylamine et d'hexafluorophosphonate de benzotriazinyl-1 oxy-tris-(diméthylamino) phosphonium, à une température voisine de 25°C.

L'acide correspondant peut être obtenu par hydrolyse d'un composé de formule (I).

Cette hydrolyse s'effectue généralement au sein d'un solvant chloré tel que le chloroforme ou le chlorure de méthylène, au moyen d'acide trifluoroacétique, à la température d'ébullition du solvant.

Les composés de formule (I) pour lesquels R₁ représente un radical phényle substitué par un radical amino peuvent également être préparés par réduction du dérivé nitré correspondant.

Cette réduction peut être effectuée notamment au moyen de fer en poudre et d'acide chlorhydrique au sein d'un mélange alcool-eau, à la température d'ébullition du milieu réactionnel.

Il est entendu pour l'homme du métier que, pour la mise en oeuvre des procédés selon l'invention décrits précédemment, il peut être nécessaire d'introduire des groupes protecteurs des fonctions amino afin d'éviter des réactions secondaires. Ces fonctions peuvent par exemple être bloquées sous forme de trifluorométhylacétamide puis régénérées par action de méthanol ammoniacal après avoir mis en oeuvre le procédé selon l'invention.

Les énantiomères des composés de formule (I) contenant au moins un site asymétrique peuvent être obtenus par dédoublement des racémiques par exemple par chromatographie sur colonne chirale selon W.H. PIRCKLE et coll., asymetric synthesis, vol.1, Academic Press (1983) ou par synthèse à partir des précurseurs chiraux.

Les composés de formule (I) peuvent être purifiés par les méthodes connues habituelles, par exemple par cristallisation, chromatographie ou extractions.

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, de la maladie de Parkinson, de la diskinésie tardive, du syndrôme du colon irritable, de la pancréatite aiguë, des ulcères et des désordres de la motilité intestinale, de certaines tumeurs de l'oesophage inférieur, du colon et de l'intestin et comme régulateur de l'appétit.Ces composés ont également un effet de potentialisation sur l'activité analgésique des médicaments narcotiques et non narcotiques.

L'affinité des composés de formule (I) pour les récepteurs CCK a été déterminée selon une technique inspirée de celle de A. SAITO et coll. (J. Neuro.Chem.37, 483-490(1981)) au niveau du cortex cérébral et au niveau du pancréas.

Dans ces tests, la CI₅₀ des composés de formule (I) est généralement inférieure ou égale à 1 000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 16-23 (1989) ; REYFELD et coll., Am. J. Physiol., 240, G255-266 (1981) ; BEINFELD et coll, Neuropeptides, 3, 411-427 (1983)).

Les composés de formule (I) présentent une toxicité faible. Leur DL₅₀ est généralement supérieure à 40 mg/kg par voie sous cutanée chez la souris.

D'un intérêt particulier sont les composés de formule (I) pour lesquels :
- R₁ représente un atome d'hydrogène ou un radical alcoxycarbonyle ou phényle éventuellement substitué par un ou plusieurs substituants choisis parmi les atomes d'halogène et les radicaux alcoxy, nitro et amino,
- R₂ représente un radical alkyle, polyfluoroalkyle, cinnamyle, cycloalkyle non substitué ou substitué par un ou plusieurs radicaux alkyle,
- R₃ représente un radical phényle substitué par un radical alkyle ou par un atome d'halogène, un radical quinolyle
- m est égal à 0 ou 1. Sont particulièrement intéressants les composés suivants:
- (amino-4 phényl)-2 {[(méthyl-3 phényl)-3 uréido]-2 N-phényl-acétamido}-4 acétate de tert-butyle-(RS)
- (chloro-3 phényl)-2 {[(méthyl-3 phényl)-3 uréido]-2 N-phényl-acétamido}-2 acétate de tert-butyle-(RS)
- (chloro-2 phényl)-2 {[(méthyl-3 phényl)-3 uréido]-2 N-phényl-acétamido}-2 acétate de tert-butyle-(RS)
- (fluoro-3 phényl)-2 {[(méthyl-3 phényl)-3 uréido]-2 N-phényl-acétamido}-2 acétate de tert-butyle-(RS).

Les exemples suivants illustrent l'invention sans la limiter.

### EXEMPLE 1

Une suspension de 3,77 g d'anilino-2 phényl-2 acétate d'hexafluoro-1,1,1,3,3,3 propyl-2-(RS) et de 2,08 g d'acide [(méthyl-3 phényl)-3 uréido]-2 acétique dans 50 cm3 de dichloro-1,2 éthane anhydre est chauffé à reflux. On ajoute alors 1,18 g de chlorure de thionyle en maintenant le reflux jusqu'à la fin du dégagement gazeux. Le mélange réactionnel est alors versé dans 30 cm3 d'une solution aqueuse saturée d'hydrogénocarbonate de sodium puis on ajoute 50 cm3 de chlorure de méthylène. La phase organique est lavée par 50 cm3 d'eau, séchée sur sulfate de magnésium et concentrée à sec sous pression réduite (2,7 kPa) à 40°C. Le produit est purifié par chromatographie sur 100 g de silice (0,063-0,200 mm) contenus dans une colonne de 3,5 cm de diamètre [éluant : chlorure de méthylène-méthanol (99-1 en volumes)] en recueillant des fractions de 50 cm3. Les fractions 14 à 22 sont réunies et concentrées à sec sous pression réduite (2,7 kPa) à 40°C. On obtient après recristallisation dans l'acétonitrile 2,5 g de {[(méthyl-3 phényl)-3 uréido]-2 N-phényl-acétamido)-2 phénylacétate d'hexafluoro-1,1,1,3,3,3 propyl-2-(RS) fondant à 160°C.

L'anilino-2 phényl-2 acétate d'hexafluoro-1,1,1,3,3,3 propyl-2-(RS) peut être préparé de la manière suivante : à une solution de 10,24 g d'aniline dans 150 cm3 d'acétonitrile on ajoute 16,1 g d'a-bromophénylacétate d'hexafluoro-1,1,1,3,3,3 propyl-2-(RS) et le mélange est agité à reflux pendant 3 heures. Le produit insoluble est séparé par filtration et le filtrat est concentré à sec sous pression réduite (2,7 kPa) à 40°C. Le produit obtenu est purifié par chromatographie sur 100 g de silice (0,063-0,200 mm) contenus dans une colonne de 2,7 cm de diamètre [éluant cyclohexane-acétate d'éthyle (90-10 en volumes)] en recueillant des fractions de 40 cm3. Les fractions 5 à 18 sont réunies et concentrées à sec sous pression réduite (2,7 kPa) à 40°C. On obtient ainsi 12 g d'anilino-2 phényle-2 acétate d'hexafluoro-1,1,1,3,3,3 propyl-2-(RS) sous forme d'une huile utilisée telle quelle dans les synthèses ultérieures.

L'α-bromophénylacétate d'hexafluoro-1,1,1,3,3,3 propyl-2-(RS) peut être préparé de la manière suivante : à une solution de 10 g d'hexafluoro-1,1,1,3,3,3 propanol-2 et de 6,6 g de triéthylamine dans 80 cm3 d'oxyde de diéthyle maintenueà une température voisine de 5°C, on ajoute en 20 minutes, 14 g de chlorure d'α-bromophénylacétyle puis le mélange est agité pendant 2 heures à une température voisine de 25°C. Le produit insoluble est séparé par filtration et le filtrat est lavé successivement par 20 cm3 d'une solution aqueuse d'acide chlorhydrique 4N, 20 cm3 d'une solution aqueuse saturée d'hydrogénocarbonate de sodium, 25 cm3 d'eau puis séché sur sulfate de magnésium et concentré à sec sous pression réduite (2,7 kPa) à 30°C. On obtient ainsi 16,1 g d'α-bomophénylacétate d'hexafluoro-1,1,1,3,3,3 propyl-2-(RS) sous forme d'une huile utilisée telle quelle dans les synthèses ultérieures.

L'acide [(méthyl-3 phényl)-3 uréido]-2 acétique peut être préparé de la manière suivante : à une solution de 30 g de glycine et de 53 g d'hydrogénocarbonate de sodium dans 600 cm3 d'eau, on ajoute en 15 minutes 53 g d'isocyanate de méthyl-3 phényle. Le mélange réactionnel est agité 4 heures à une température voisine de 25°C puis lavé par 200 cm3 d'acétate d'éthyle et acidifié à pH 1 avec 200 cm3 d'une solution d'acide chlorhydrique 4N. Le produit est séparé par filtration, lavé à l'eau et séché à l'air. On obtient ainsi 72 g d'acide [(méthyl-3 phényl)-3 uréido]-2 acétique fondant à 208°C.

### Exemple 2

En opérant d'une manière analogue à celle décrite à l'exemple 1, mais à partir de 4,8 g d'anilino-2 acétate de méthyl-1 cyclopentyle, de 4,1 g d'acide [(méthyl-3 phényl)-3 uréido]-2 acétique et de 1,4 cm3 de chlorure de thionyle, on obtient après recristallisation dans un mélange acétonitrile-oxyde de diisopropyle (40-60 en volumes), 0,6 g de {[(méthyl-3 phényl)-3 uréido]-2 N-phényl-acétamido}-2 acétate de méthyl-1 cyclopentyle fondant à 160°C.

L'anilino-2 acétate de méthyl-1 cyclopentyle peut être préparé d'une manière analogue à celle décrite à l'exemple 1 pour la préparation de l'anilino-2 phényl-2 acétate d'hexafluoro-1,1,1,-3,3,3 propyl-2-(RS), mais à partir de 5,7 g de bromoacétate de méthyl-1 cyclopentyle et de 4,6 g d'aniline. Le produit est purifié par chromatographie sur 70 g de silice (0,063-0,200 mm) contenus dans une colonne de 2 cm de diamètre [éluant : cyclohexane-acétate d'éthyle (70-30 en volumes)] en recueillant des fractions de 20 cm3. Les fractions 3 à 6 sont réunies et concentrées à sec sous pression réduite (2,7 kPa) à 40°C. On obtient ainsi 4,8 g d'anilino-2 acétate de méthyl-1 cyclopentyle sous forme d'une huile utilisée telle quelle dans les synthèses ultérieures.

Le bromoacétate de méthyl-1 cyclopentyle peut être préparé de la manière suivante : à une solution de 10 g de méthyl-1 cyclopentanol et de 12 g de N,N-diméthyl aniline dans 100 cm3 de dichloro-1,2 éthane, maintenue à une température voisine de 0°C, on ajoute en 5 minutes 8,4 g de bromure de bromoacétyle. Le mélange réactionnel est agité à une température voisine de 25°C pendant 16 heures, lavé successivement par 2 fois 25 cm3 d'une solution aqueuse d'acide chlorhydrique 4N, 25 cm3 d'eau, 25 cm3 d'une solution aqueuse saturée d'hydrogénocarbonate de sodium. La phase organique est séchée sur sulfate de magnésium et concentrée à sec sous pression réduite (2,7 kPa) à 40°C. Le produit obtenu est purifié par chromatographie sur 120 g de silice (0,063-0,200 mm) contenus dans une colonne de 3,0 cm de diamètre (éluant : dichlorométhane) en recueillant des fractions de 20 cm3. Les fractions 2 à 4 sont réunies et concentrées à sec sous pression réduite (2,7 kPa) à 40°C. On obtient ainsi, 5,7 g de bromoacétate de méthyl-1 cyclopentyle sous forme d'une huile utilisée telle quelle dans les synthèses ultérieures.

### Exemple 3

On opère d'une manière analogue à celle décrite à l'exemple 1, mais à partir de 4,8 g d'anilino-2 acétate de méthyl-2 cyclohexyle-(1RS,2SR), de 4,0 g d'acide [(méthyl-3 phényl)-3 uréido]-2 acétique et de 1,4 cm3 de chlorure de thionyle. On obtient, après recristallisation dans un mélange acétonitrile-oxyde de diisopropyle (40-60 en volumes), 2,0 g de {[(méthyl-3 phényl)-3 uréido]-2 N-phényl-acétamido}-2 acétate de méthyl-2 cyclohexyle-(1RS,2SR) fondant à 140°C.

L'anilino-2 acétate de méthyl-2 cyclohexyle-(1RS,2SR) peut être préparé d'une manière analogue à celle décrite à l'exemple 1, pour la préparation de l'anilino-2 phényl-2 acétate d'hexafluoro-1,1,1,3,3,3 propyl-2-(RS), mais à partir de 4,7 g de bromoacétate de méthyl-2 cyclohexyle-(1RS,2SR) et de 3,7 g d'aniline. Le produit obtenu est purifié par chromatographie sur 40 g de silice (0,063-0,200 mm) contenus dans une colonne de 1,5 cm de diamètre [éluant : cyclohexane-acétate d'éthyle (70-30 en volumes)] en recueillant des fractions de 20 cm3. Les fractions 3 à 8 sont réunies et concentrées à sec sous pression réduite (2,7 kPa) à 40 °C. On obtient ainsi 4,8 g d'anilino-2 acétate de méthyl-2 cyclohexyle-(1RS,2SR) sous forme d'une huile utilisée telle quelle dans les synthèses ultérieures.

Le bromoacétate de méthyl-2 cyclohexyle-(1RS,2SR) peut être préparé d'une manière analogue à celle décrite à l'exemple 2, pour la préparation du bromoacétate de méthyl-1 cyclopentyle, mais à partir de 6,8 g de méthyl-2 cyclohexanol-(1RS, 2SR), de 7,3 g de N,N-diméthyl aniline et de 5 g de bromure de bromoacétyle. Le produit obtenu est purifié par chromatographie sur 40 g de silice (0,063-0,200 mm) contenus dans une colonne de 1,5 cm de diamètre (éluant : dichlorométhane) en recueillant des fractions de 20 cm3. Les fractions 2 à 4 sont réunies et concentrées à sec sous pression réduite (2,7 kPa) à 40 °C. On obtient ainsi 4,7 g de bromoacétate de méthyl-2 cyclohexyle-(1RS,2SR) sous forme d'une huile utilisée telle quelle dans les synthèses ultérieures.

### Exemple 4

En opérant d'une manière analogue à celle décrite à l'exemple 1, mais à partir de 3,0 g d'anilino-2 acétate de méthyl-1 cyclohexyle, de 2,5 g d'acide [(méthyl-3 phényl)-3 uréido]-2 acétique et de 0,9 cm3 de chlorure de thionyle. Le produit obtenu est purifié par chromatographie sur 40 g de silice (0,063-0,200 mm) contenus dans une colonne de 1,5 cm de diamètre [éluant cyclohexane-acétate d'éthyle (70-30 en volumes)] en recueillant des fractions de 20 cm3. Les fractions 3 à 8 sont réunies et concentrées à sec sous pression réduite (2,7 kPa) à 40 °C. On obtient, après recristallisation dans un mélange acétonitrile-oxyde de diisopropyle (50-50 en volumes), 0,6 g de {[(méthyl-3 phényl)-3 uréido]-2 N-phényl-acétamido}-2 acétate de méthyl-1 cyclohexyle fondant à 192°C.

L'anilino-2 acétate de méthyl-1 cyclohexyle peut être préparé d'une manière analogue à celle décrite à l'exemple 1, pour la préparation de l'anilino-2 phényl-2 acétate d'hexafluoro-1,1,1 3,3,3 propyl-2-(RS), mais à partir de 3,8 g de bromoacétate de méthyl-1 cyclohexyle et de 3,0 g d'aniline. Le produit obtenu est purifié par chromatographie sur 80 g de silice (0,063-0,200 mm) contenus dans une colonne de 2 cm de diamètre [éluant cyclohexane-acétate d'éthyle (70-30 en volumes)] en recueillant des fractions de 20 cm3. Les fractions 2 à 6 sont réunies et concentrées à sec sous pression réduite (2,7 kPa) à 40 °C. On obtient ainsi 3,0 g d'anilino-2 acétate de méthyl-1 cyclohexyle sous forme d'une huile utilisée telle quelle dans les synthèses ultérieures.

Le bromoacétate de méthyl-1 cyclohexyle peut être préparé d'une manière analogue à celle décrite à l'exemple 2, pour la préparation du bromoacétate de méthyl-1 cyclopentyle, mais à partir de 6,8 g de méthyl-1 cyclohexanol, de 7,3 g de N,N-diméthyl aniline et de 5,0 g de bromure de bromoacétyle. Le produit obtenu est purifié par chromatographie sur 40 g de silice (0,063-0,200 mm) contenus dans une colonne de 1,5 cm de diamètre (éluant dichlorométhane) en recueillant des fractions de 20 cm3. Les fractions 2 à 5 sont réunies et concentrées à sec sous pression réduite (2,7 kPa) à 40 °C. On obtient ainsi, 3,8 g de bromoacétate de méthyl-1 cyclohexyle sous forme d'une huile utilisée telle quelle dans les synthèses ultérieures.

### Exemple 5

On opère d'une manière analogue à celle décrite à l'exemple 1, mais à partir de 3,3 g d'anilino-2 acétate de cinnamyle, de 2,6 g d'acide [(méthyl-3 phényl)-3 uréido]-2 acétique et de 0,9 cm3 de chlorure de thionyle. Le produit obtenu est purifié par chromatographie sur 60 g de silice (0,063-0,200 mm) contenus dans une colonne de 2,0 cm de diamètre [éluant : cyclohexane-acétate d'éthyle (50-50 en volumes)] en recueillant des fractions de 20 cm3. Les fractions 4 à 8 sont réunies et concentrées à sec sous pression réduite (2,7 kPa) à 40°C. On obtient, après recristallisation dans l'acétonitrile, 1,4 g de {[(méthyl-3 phényl)-3 uréido]-2 N-phényl-acétamido}-2 acétate de cinnamyle fondant à 158°C.

L'anilino-2 acétate de cinnamyle peut être préparé d'une manière analogue à celle décrite à l'exemple 1, pour la préparation de l'anilino-2 phényl-2 acétate d'hexafluoro-1,1,1,3,3,3 propyl-2-(RS), mais à partir de 3,8 g de bromoacétate de cinnamyle et de 2,8g d'aniline. Le produit obtenu est purifié par chromatographie sur 50 g de silice (0,063-0,200 mm) contenus dans une colonne de 2 cm de diamètre [éluant : cyclohexane-acétate d'éthyle (70-30 en volumes)] en recueillant des fractions de 20 cm3. Les fractions 3 à 6 sont réunies et concentrées à sec sous pression réduite (2,7 kPa) à 40 °C. On obtient ainsi 3,4 g d'anilino-2 acétate de cinnamyle sous forme d'une huile utilisée telle quelle dans les synthèses ultérieures.

Le bromoacétate de cinnamyle peut être préparé d'une manière analogue à celle décrite à l'exemple 1, pour la préparation de l'α-bromophénylacétate d'hexafluoro-1,1,1,3,3,3, propyl-2-(RS) mais à partir de 3,9 g d'alcool cinnamique, de 2,7 g de triéthylamine et de 5,0 g de bromure de bromoacétyle. On obtient ainsi 3,3g de bromoacétate de cinnamyle sous forme d'une huile utilisée telle quelle dans les synthèses ultérieures.

### Exemple 6

On opère d'une manière analogue à celle décrite à l'exemple 1, mais à partir de 2,3 g d'anilino-2 acétate de trifluoro-2,2,2 éthyle, de 2,1 g d'acide [(méthyl-3 phényl)-3 uréido]-2 acétique et de 0,7 cm3 de chlorure de thionyle. Le produit obtenu est purifié par chromatographie sur 40 g de silice (0,063-0,200 mm) contenus dans une colonne de 1,5 cm de diamètre [éluant : chlorure de méthylène-éthanol (98-2 en volumes)] en recueillant des fractions de 10 cm3. Les fractions 5 à 8 sont réunies et concentrées à sec sous pression réduite (2,7 kPa) à 40 °C. On obtient, après recristallisation dans l'oxyde de diisopropyle, 0,6 g de {[(méthyl-3 phényl)-3 uréido]-2 N-phényl-acétamido}-2 acétate de trifluoro-2,2,2 éthyle fondant à 172°C.

L'anilino-2 acétate de trifluoro-2,2,2 éthyle peut être préparé d'une manière analogue à celle décrite à l'exemple 1, pour la préparation de l'anilino-2 phényl-2 acétate d'hexafluoro-1,1,1, 3,3,3 propyl-2-(RS), mais à partir de 3,5 g de bromoacétate de trifluoro-2,2,2 éthyle et de 2,9 g d'aniline. Le produit obtenu est purifié par chromatographie sur 50 g de silice (0,063-0,200 mm) contenus dans une colonne de 2 cm de diamètre [éluant cyclohexane-acétate d'éthyle (50-50 en volumes)] en recueillant des fractions de 20 cm3. Les fractions 3 à 5 sont réunies et concentrées à sec sous pression réduite (2,7 kPa) à 40 °C. On obtient ainsi 2,3 g d'anilino-2 acétate de trifluoro-2,2,2 éthyle sous forme d'une pâte utilisée telle quelle dans les synthèses ultérieures.

Le bromoacétate de trifluoro-2,2,2 éthyle peut être préparé d'une manière analogue à celle décrite à l'exemple 1, pour la préparation de l'α-bromophénylacétate d'hexafluoro-1,1,1,3,3,3 propyl-2-(RS), mais à partir de 2,5 g de trifluoro-2,2,2 éthanol, de 2,7 g de triéthylamine et de 5,0 g de bromure de bromoacétyle. On obtient ainsi 2,9 g de bromoacétate de trifluoro-2,2,2 éthyle sous forme d'une huile utilisée telle quelle dans les synthèses ultérieures.

### Exemple 7

On opère d'une manière analogue à celle décrite à l'exemple 1, mais à partir de 1,7 g d'anilino-2 acétate de pentyl-3, de 1,5 g d'acide [(méthyl-3 phényl)-3 uréido]-2 acétique et de 0,9 g de chlorure de thionyle. Le produit obtenu est purifié par chromatographie sur 100 g de silice (0,063-0,200 mm) contenus dans une colonne de 2,5 cm de diamètre [éluant : cyclohexane-acétate d'éthyle (70-30 en volumes)] en recueillant des fractions de 30 cm3. Les fractions 19 à 32 sont réunies et concentrées à sec sous pression réduite (2,7 kPa) à 40 °C. On obtient, après recristallisation dans l'oxyde de diisopropyle, 0,5 g de {[(méthyl-3 phényl)-3 uréido]-2 N-phényl-acétamido}-2 acétate de pentyl-3 fondant à 118°C.

L'anilino-2 acétate de pentyl-3 peut être préparé d'une manière analogue à celle décrite à l'exemple 1, pour la préparation de l'anilino-2 phényl-2 acétate de d'hexafluoro-1,1,1,3,3,3 propyl-2-(RS), mais à partir de 5,0 g de bromoacétate de pentyl-3 et de 4,5 g d'aniline. Le produit obtenu est purifié par chromatographie sur 100 g de silice (0,063-0,200 mm) contenus dans une colonne de 3,5 cm de diamètre (éluant : dichlorométhane) en recueillant des fractions de 50 cm3. Les fractions 5 à 13 sont réunies et concentrées à sec sous pression réduite (2,7 kPa) à 40°C. On obtient ainsi 8,6 g d'anilino-2 acétate de pentyl-3 sous forme d'une huile utilisée telle quelle dans les synthèses ultérieures.

Le bromoacétate de pentyl-3 peut être préparé d'une manière analogue à celle décrite à l'exemple 1, pour la préparation de l'α-bromophénylacétate d'hexafluoro-1,1,1,3,3,3 propyl-2-(RS), mais à partir de 3,8 g de pentanol-3, de 4,7 g de triéthylamine et de 8,5 g de bromure de bromoacétyle. On obtient ainsi 5,0 g de bromoacétate de pentyl-3 sous forme d'une huile utilisée telle quelle dans les synthèses ultérieures.

### Exemple 8

On opère comme à l'exemple 1, mais à partir de 2,1 g d'acide [(méthyl-3 phényl)-3 uréido]-2 acétique, de 3,14 g d'anilino-2 (méthoxy-4 phényl)-2 acétate de tert-butyle-(RS) et de 0,72 cm3 de chlorure de thionyle. Le résidu obtenu est purifié par chromatographie sur 100 g de silice (0,04-0,063 mm) contenus dans une colonne de 3 cm de diamètre [éluant: cyclohexane-acétate d'éthyle (70-30 en volumes)] en utilisant une surpression de 40 kPa d'azote et en recueillant des fractions de 10 cm3. Les fractions 12 à 18 sont réunies, concentrées à sec sous pression réduite (2,7 kPa) à 40°C et le résidu obtenu est recristallisé dans un mélange oxyde de diisopropyle-acétonitrile (50-50 en volumes). On obtient ainsi 2,3 g de (méthoxy-4 phényl)-2 {[(méthyl-3 phényl)-3 uréido]-2 N-phényl-acétamido}-2 acétate de tert-butyle-(RS) fondant à 206°C.

L'anilino-2 (méthoxy-4 phényl)-2 acétate de tert-butyle-(RS) peut être préparé de la façon suivante: à une solution de 20 g de bromo-2 (méthoxy-4 phényl)-2 acétate de tert-butyle-(RS) dans 100 cm3 d'acétonitrile, on ajoute 12,1 cm3 d'aniline et le mélange réactionnel est maintenu à reflux pendant 5 heures puis pendant 20 heures à une température voisine de 20°C. Le produit insoluble est séparé par filtration et le filtrat est concentré à sec sous pression réduite (2,7 kPa). Le produit obtenu est purifié par chromatographie sur 250 g de silice (0,04-0,063 mm) contenus dans une colonne de 4,2 cm de diamètre [éluant : acétate d'éthyle-cyclohexane (10-90 en volumes)] en utilisant une surpression de 40 kPa d'azote et en recueillant des fractions de 10 cm3. Les fractions 6 à 17 sont réunies, concentrées à sec sous pression réduite (2,7 kPa) à 40°C et le résidu obtenu est cristallisé dans 150 cm3 d'éther de pétrole. On obtient ainsi 12 g d'anilino-2 (méthoxy-4 phényl)-2 acétate de tert-butyle-(RS) fondant à 99°C.

Le bromo-2 (méthoxy-4 phényl)-2 acétate de tert-butyle-(RS) peut être préparé de la façon suivante: à une suspension de 15 g de (méthoxy-4 phényl)-2 acétate de tert-butyle dans 74 cm3 de tétrachlorure de carbone, on ajoute 13,2 g de N-bromo succinimide et 0,1 g d'azo-bis-isobutyronitrile. Le mélange réactionnel est maintenu à reflux pendant 8 heures puis à une température voisine de 20°C pendant 20 heures. Le produit insoluble est séparé par filtration et le filtrat est concentré à sec sous pression réduite (2,7 kPa). On obtient ainsi 20 g de bromo-2 (méthoxy-4 phényl)-2 acétate de tert-butyle-(RS) à l'état d'huile utilisée telle quelle dans les synthèses ultérieures.

Le (méthoxy-4 phényl)-2 acétate de tert-butyle peut être préparé de la façon suivante: à une solution de 31 cm3 de N,N-diméthylaniline dans 41,3 cm3 de tertiobutanol, on ajoute 18 g de chlorure de (méthoxy-4 phényl)-2 acétyle. Le mélange est agité pendant 20 heures à une température voisine de 20°C puis versé dans 400 cm3 d'oxyde de diéthyle et 200 cm3 d'eau. La phase organique est séparée, lavée par 3 fois 200 cm3 d'eau, 3 fois 200 cm3 d'une solution aqueuse d'acide chlorhydrique 2N et enfin par 3 fois 200 cm3 d'eau, puis séchée sur sulfate de magnésium et concentrée à sec sous pression réduite (2,7 kPa). Le produit obtenu est purifié par chromatographie sur 350 g de silice (0,04-0,063 mm) contenus dans une colonne de 7 cm de diamètre [éluant : acétate d'éthyle-cyclohexane (10-90 en volumes)] en utilisant une surpression de 40 kPa d'azote et en recueillant des frations de 10 cm3. Les fractions 1 à 18 sont réunies et concentrées à sec sous pression réduite (2,7 kPa) à 40°C. On obtient ainsi 15 g de (méthoxy-4 phényl)-2 acétate de tert-butyle à l'état d'huile utilisée telle quelle dans les synthèses ultérieures.

Le chlorure de (méthoxy-4 phényl)-2 acétyle peut être préparé selon la méthode décrite par H. YAMAGUCHI, Yakugaku Zaski, 78, 733 (1958).

### Exemple 9

On opère comme à l'exemple 8, mais à partir de 2,1 g d'acide [(méthyl-3 phényl)-3 uréido]-2 acétique, de 3,18 g d'anilino-2 (chloro-4 phényl)-2 acétate de tert-butyle-(RS) et de 0,72 cm3 de chlorure de thionyle. Le produit obtenu est purifié par chromatographie sur 250 g de silice (0,04-0,063 mm) contenus dans une colonne de 4,2 cm de diamètre [éluant :acétate d'éthyle-cyclohexane (30-70 en volumes)] en utilisant une surpression de 40 kPa d'azote et en recueillant des fractions de 10 cm3. Les fractions 8 à 17 sont réunies, concentrées à sec sous pression réduite (2,7 kPa) à 40°C et le résidu obtenu est recristallisé dans l'oxyde de diisopropyle. On obtient ainsi 2,6 g de (chloro-4 phényl)-2 {[(méthyl-3 phényl)-3 uréido]-2 N-phényl-acétamido}-2 acétate de tert-butyle-(RS) fondant à 167°C.

L'anilino-2 (chloro-4 phényl)-2 acétate de tert-butyle-(RS) peut être préparé d'une manière analogue à celle décrite à l'exemple 8, pour la préparation de l'anilino-2 (méthoxy-4 phényl)-2 acétate de tert-butyle-(RS), mais à partir de 15,3 g de bromo-2 (chloro-4 phényl)-2 acétate de tert-butyle-(RS) et de 9,1 cm3 d'aniline dans 150 cm3 d'acétonitrile. On obtient ainsi 5,2 g d'anilino-2 ( chloro-4 phényl)-2 acétate de tert-butyle-(RS) fondant à 98°C.

Le bromo-2 (chloro-4 phényl)-2 acétate de tert-butyle-(RS) peut être préparé de la façon suivante: à une solution de 63 cm3 de tertiobutanol et de 46,9 cm3 de N,N-diméthylaniline on ajoute en 1 heure, 40 g de chlorure de bromo-2 (chloro-4 phényl)-2 acétyle-(RS). Le mélange est maintenu à une température voisine de 20°C pendant 20 heures puis versé dans 400 cm3 d'oxyde de diéthyle et 200 cm3 d'eau. La phase organique est séparée, lavée par 3 fois 200 cm3 d'eau, 3 fois 200 cm3 d'une solution aqueuse d'acide chlorhydrique 2N et enfin par 3 fois 200 cm3 d'eau, puis séchée sur sulfate de magnésium et concentrée à sec sous pression réduite (2,7 kPa). On obtient ainsi 32,5 g de bromo-2 (chloro-4 phényl)-2 acétate de tert-butyle-(RS) à l'état d'huile utilisée telle quelle dans les synthèses ultérieures.

Le chlorure de bromo-2 (chloro-4 phényl)-2 acétyle-(RS) peut être préparé de la façon suivante: une solution de 25,6 g d'acide (chloro-4 phényl)-2 acétique et de 18,6 g de chlorure de thionyle est agitée pendant 3 heures à reflux jusqu'à la fin du dégagement gazeux. Le mélange est refroidi à une température voisine de 30°C. On ajoute 8,45 cm3 de brome puis on chauffe pendant 5 heures à reflux. Le mélange réactionnel est concentré à sec sous pression réduite (2,7kPa) à 45°C. On obtient ainsi 40 g de chlorure de bromo-2 (chloro-4 phényl)-2 acétyle-(RS) utilisé tel quel pour l'étape suivante.

### Exemple 10

On opère comme à l'exemple 1, mais à partir de 2,1 g d'acide [(méthyl-3 phényl)-3 uréido]-2 acétique, de 3,52 g d' anilino-2 (dichloro-3,4 phényl)-2 acétate de tert-butyle-(RS) et de 0,72 cm3 de chlorure de thionyle. Le produit obtenu est purifié par chromatographie sur 100 g de silice (0,04-0,063 mm) contenus dans une colonne de 3 cm de diamètre [éluant : acétate d'éthylecyclohexane (30-70 en volumes)] en utilisant une surpression de 40 kPa d'azote et en recueillant des fractions de 10 cm3. Les fractions 13 à 24 sont réunies, concentrées à sec sous pression réduite (2,7 kPa) à 40°C et le résidu obtenu est recristallisé dans l'oxyde de diisopropyle. On obtient ainsi 2,6 g de (dichloro-3,4 phényl)-2 {[(méthyl-3 phényl)-3 uréido]-2 N-phényl-acétamido}-2 acétate de tert-butyle-(RS) fondant à 176°C

L'anilino-2 (dichloro-3,4 phényl)-2 acétate de tert-butyle-(RS) peut être préparé d'une manière analogue à celle décrite à l'exemple 8, pour la préparation de l'anilino-2 (méthoxy-4 phényl)-2 acétate de tert-butyle-(RS), mais à partir de 17 g de bromo-2 (dichloro-3,4 phényl)-2 acétate de tert-butyle-(RS) et de 9,1 cm3 d'aniline dans 100 cm3 d'acétonitrile. On obtient ainsi 9,0 g d'anilino-2 (dichloro-3,4 phényl)-2 acétate de tert-butyle-(RS) fondant à 94°C.

Le bromo-2 (dichloro-3,4 phényl)-2 acétate de tert-butyle-(RS) peut être préparé d'une manière analogue à celle décrite à l'exemple 9 pour la préparation du bromo-2 (chloro-4 phényl)-2 acétate de tert-butyle-(RS) mais à partir de 38 g de chlorure de bromo-2 (dichloro-3,4 phényl)-2 acétyle-(RS), de 53,2 cm3 de tertiobutanol et de 39,7 cm3 de N,N-diméthylaniline. Le produit obtenu est purifié par chromatographie sur 250 g de silice (0,04-0,063 mm) contenus dans une colonne de 4,2 cm de diamètre [éluant : acétate d'éthyle-cyclohexane (10-90 en volumes)] en utilisant une surpression de 40 kPa d'azote et en recueillant des fractions de 10 cm3. Les fractions 5 à 12 sont réunies et concentrées à sec sous pression réduite (2,7 kPa) à 40°C. Par cristallisation du résidu obtenu dans 200 cm3 d'éther de pétrole, on obtient 20 g de bromo-2 (dichloro-3,4 phényl)-2 acétate de tert-butyle-(RS) fondant à 88°C.

Le chlorure de bromo-2 (dichloro-3,4 phényl)-2 acétyle-(RS) peux être préparé selon la méthode décrite à l'exemple 9, pour la préparation du chlorure de bromo-2 (chloro-4 phényl)-2 acétyle-(RS), mais à partir de 25 g d'acide (dichloro-3,4 phényl)-2 acétique, de 14,9 cm3 de chlorure de thionyle et de 7,7 cm3 de brome. On obtient ainsi 38 g de chlorure de bromo-2 (dichloro-3,4 phényl)-2 acétyle-(RS) utilisé tel quel pour l'étape suivante.

### Exemple 11

On opère comme à l'exemple 1, mais à partir de 4,19 g d'acide [(méthyl-3 phényl)-3 uréido]-2 acétique, de 6,57 g d' anilino-2 (nitro-4 phényl)-2 acétate de tert-butyle-(RS) dans 100 cm3 de dichloro-1,2 éthane anhydre et de 1,42 cm3 de chlorure de thionyle. Le produit obtenu est purifié par chromatographie sur 250 g de silice (0,04-0,063 mm) contenus dans une colonne de 4,2 cm de diamètre [éluant: acétate d'éthyle-cyclohexane (30-70 en volumes)] en utilisant une surpression de 40 kPa d'azote et en recueillant des fractions de 10 cm3. Les fractions 25 à 39 sont réunies, concentrées à sec sous pression réduite (2,7 kPa) à 40°C et le résidu obtenu est recristallisé dans l'acétonitrile. On obtient ainsi 3 g de {[(méthyl-3 phényl)-3 uréido]-2 N-phényl-acétamido}-2 (nitro-4 phényl)-2 acétate de tert-butyle-(RS) fondant à 212°C.

L'anilino-2 (nitro-4 phényl)-2 acétate de tert-butyle-(RS) peut être préparé d'une manière analogue à celle décrite à l'exemple 8, pour la préparation de l'anilino-2 (méthoxy-4 phényl)-2 acétate de tert-butyle-(RS), mais à partir de 23,7 g de bromo-2 (nitro-4 phényl)-2 acétate de tert-butyle-(RS) et de 13,7 cm3 d'aniline dans 100 cm3 d'acétonitrile. Le produit obtenu est purifié par chromatographie sur 250 g de silice (0,04-0,063 mm) contenus dans une colonne de 4,2 cm de diamètre [éluant: acétate d'éthyle-cyclohexane (20-80 en volumes)] en utilisant une surpression de 40 kPa d'azote et en recueillant des fractions de 20 cm3. Les fractions 10 à 18 sont réunies et concentrées à sec sous pression réduite (2,7 kPa) à 40°C. On obtient ainsi 15 g d'anilino-2 (nitro-4 phényl)-2 acétate de tert-butyle-(RS) à l'état d'huile utilisée telle quelle dans les synthèses ultérieures.

Le bromo-2 (nitro-4 phényl)-2 acétate de tert-butyle-(RS) peut être préparé d'une manière analogue à celle décrite à l'exemple 8, pour la préparation du bromo-2 (chloro-4 phényl)-2 acétate de tert-butyle-(RS) mais à partir de 56g de chlorure de (nitro-4 phényl)-2 bromacétyle-(RS), de 84,7 cm3 de tertiobutanol et de 63 cm3 de N,N-diméthylaniline. Le produit obtenu est purifié par chromatographie sur 250 g de silice (0,04-0,063 mm) contenus dans une colonne de 4,2 cm de diamètre [éluant : acétate d'éthyle-cyclohexane (10-90 en volumes)] en utilisant une surpression de 40 kPa d'azote et en recueillant des fractions de 20 cm3. Les fractions 7 à 20 sont réunies et concentrées à sec sous pression réduite (2,7 kPa) à 40°C. On obtient ainsi 37 g de bromo-2 (nitro-4 phényl)-2 acétate de tert-butyle-(RS) à l'état d'huile utilisée telle quelle dans les synthèses ultérieures.

Le chlorure de bromo-2 (nitro-4 phényl)-2 acétyle-(RS) peut être préparé selon la méthode décrite à l'exemple 9, pour la préparation du chlorure de bromo-2 (chloro-4 phényl)-2 acétyle-(RS), mais à partir de 36,2 g d'acide (nitro-4 phényl)-2 acétique, de 24,4 cm3 de chlorure de thionyle et de 12,7 cm3 de brome. On obtient ainsi 56 g de chlorure de bromo-2 (nitro-4 phényl)-2 acétyle-(RS) utilisé tel quel pour l'étape suivante.

### Exemple 12

A une suspension de 2,1 g de {[(méthyl-3 phényl)-3 uréido]-2 N-phényl-acétamido}-2 (nitro-4 phényl)-2 acétate de tert-butyle-(RS) dans 40 cm3 d'un mélange d'éthanol et d'eau (50-50 en volumes) chauffée à reflux, on ajoute 0,68 g de fer en poudre et 0,5 cm3 d'acide chlorhydrique concentré. Le mélange réactionnel est maintenu au reflux pendant 4 heures et après refroidissement, il est alcalinisé à pH 8 avec une solution aqueuse saturée d'hydrogénocarbonate de sodium. Le produit insoluble est éliminé par filtration et le filtrat est extrait par 3 fois 100 cm3 de dichlorométhane. Les extraits organiques sont lavés par 2 fois 100 cm3 d'eau, séchés sur sulfate de magnésium et concentrés à sec sous pression réduite (2,7 kPa) à 40 °C. Le résidu huileux obtenu est purifié par chromatographie sur 100 g de silice (0,04-0,063 mm) contenus dans une colonne de 3 cm de diamètre [éluant : méthanol-dichlorométhane (1-99 en volumes)] en utilisant une surpression de 40 kPa d'azote et en recueillant des fractions de 10 cm3. Les fractions 8 à 14 sont réunies et concentrées à sec sous pression réduite (2,7 kPa) à 40°C. Après recristallisation dans l'oxyde d'isopropyle, on obtient 0,8 g d'(amino-4 phényl)-2 {[(méthyl-3 phényl)-3 uréido]-2 N-phényl-acétamido}-2 acétate de tert-butyle-(RS) fondant à 108°C.

### Exemple 13

On opère comme à l'exemple 1, mais à partir de 2,1 g d'acide [(méthyl-3 phényl)-3 uréido]-2 acétique, de 3,18 g d' anilino-2 (chloro-3 phényl)-2 acétate de tert-butyle-(RS) et de 0,72 cm3 de chlorure de thionyle. Le produit est purifié par chromatographie sur 100 g de silice (0,04-0,063 mm) contenus dans une colonne de 3 cm de diamètre [éluant : acétate d'éthyle-cyclohexane (30-70 en volumes)] en utilisant une surpression de 40 kPa d'azote et en recueillant des frations de 10 cm3. Les fractions 10 à 20 sont réunies, concentrées à sec sous pression réduite (2,7 kPa) à 40°C et le résidu obtenu est recristallisé dans un mélange oxyde d'isopropyle-cyclohexane (50-50 en volumes) pour donner 2,1 g d'un solide qui est à nouveau purifié par chromatographie sur 100 g de silice (0,04-0,063 mm) contenus dans une colonne de 3 cm de diamètre [éluant dichlorométhane-méthanol(99-1 en volumes)] en utilisant une surpression de 40 kPa d'azote et en recueillant des fractions de 10 cm3. Les fractions 11 à 26 sont réunies, concentrées à sec sous pression réduite (2,7 kPa) à 40°C et le résidu obtenu est recristallisé dans un mélange toluène-cyclohexane (20-80 en volumes). On obtient ainsi 1,7 g de (chloro-3 phényl)-2 ([(méthyl-3 phényl)-3 uréido]-2 N-phényl-acétamido}-2 acétate de tert-butyle-(RS) fondant à 100°C.

L'anilino-2 (chloro-3 phényl)-2 acétate de tert-butyle-(RS) peut être préparé d'une manière analogue à celle décrite à l'exemple 8, pour la préparation de l'anilino-2 (méthoxy-4 phényl)-2 acétate de tert-butyle-(RS), mais à partir de 15,3 g de bromo-2 (chloro-3 phényl)-2 acétate de tert-butyle-(RS) et de 9,2 cm3 d'aniline dans 100 cm3 d'acétonitrile. On obtient ainsi 4,8 g d'anilino-2 ( chloro-3 phényl)-2 acétate de tert-butyle-(RS) fondant à 96°C.

Le bromo-2 (chloro-3 phényl)-2 acétate de tert-butyle-(RS) peut être préparé d'une manière analogue à celle décrite à l'exemple 9, pour la préparation du bromo-2 (chloro-4 phényl)-2 acétate de tert-butyle-(RS) mais à partir de 31 g de chlorure de (chloro-3 phényl)-2 bromacétyle-(RS), de 49,1 cm3 de tertiobutanol et de 36,5 cm3 de N,N-diméthylaniline. Le produit obtenu est purifié par chromatographie sur 250 g de silice (0,04-0,063 mm) contenus dans une colonne de 4,2 cm de diamètre [éluant : acétate d'éthyle-cyclohexane (10-90 en volumes)] en utilisant une surpression de 40 kPa d'azote et en recueillant des fractions de 20 cm3. Les fractions 6 à 16 sont réunies et concentrées à sec sous pression réduite (2,7 kPa) à 40°C. On obtient ainsi 21 g de bromo-2 (chloro-3 phényl)-2 acétate de tert-butyle-(RS) à l'état d'huile utilisée telle quelle dans les synthèses ultérieures.

Le chlorure de bromo-2 (chloro-3 phényl)-2 acétyle-(RS) peut être préparé selon la méthode décrite à l'exemple 9, pour la préparation du chlorure de bromo-2 (chloro-4 phényl)-2 acétyle-(RS), mais à partir de 20 g d'acide (chloro-3 phényl)-2 acétique, de 14,3 cm3 de chlorure de thionyle et de 7,4 cm3 de brome. On obtient ainsi 31 g de chlorure de bromo-2 (chloro-3 phényl)-2 acétyle-(RS) utilisé tel quel pour l'étape suivante.

### Exemple 14

On opère comme à l'exemple 1, mais à partir de 2,1 g d'acide [(méthyl-3 phényl)-3 uréido]-2 acétique, de 3,2 g d'anilino-2 (chloro-2 phényl)-2 acétate de tert-butyle-(RS) et de 0,72 cm3 de chlorure de thionyle. Le produit obtenu est purifié par chromatographie sur 100 g de silice (0,04-0,063 mm) contenus dans une colonne de 3 cm de diamètre [éluant: dichlorométhane-métha-nol(99-1 en volumes)] en utilisant une surpression de 40 kPa d'azote et en recueillant des fractions de 10 cm3. Les fractions 7 à 15 sont réunies, concentrées à sec sous pression réduite (2,7 kPa) à 40°C et le résidu obtenu est recristallisé dans un mélange d'acétonitrile et d'oxyde d'isopropyle (10-90 en volumes). On obtient ainsi 2 g de (chloro-2 phényl)-2 {[(méthyl-3 phényl)-3 uréido]-2 N-phényl-acétamido}-2 acétate de tert-butyle-(RS) fondant à 181°C.

L'anilino-2 (chloro-2 phényl)-2 acétate de tert-butyle-(RS) peut être préparé d'une manière analogue à celle décrite à l'exemple 8, pour la préparation de l'anilino-2 (méthoxy-4 phényl)-2 acétate de tert-butyle-(RS), mais à partir de 15,3 g de bromo-2 (chloro-2 phényl)-2 acétate de tert-butyle-(RS) et de 9,2 cm3 d'aniline dans 75 cm3 d'acétonitrile. On obtient ainsi 7 g d'anilino-2 (chloro-2 phényl)-2 acétate de tert-butyle-(RS) fondant à 74°C.

Le bromo-2 (chloro-2 phényl)-2 acétate de tert-butyle-(RS) peut être préparé d'une manière analogue à celle décrite à l'exemple 9, pour la préparation du bromo-2 (chloro-4 phényl)-2 acétate de tert-butyle-(RS) mais à partir de 39 g de chlorure de bromo-2 (chloro-2 phényl)-2 acétyle-(RS) et de 61,6 cm3 de tertiobutanol et de 45,8 cm3 de N,N-diméthylaniline. Le produit obtenu est purifié par chromatographie sur 250 g de silice (0,04-0,063 mm) contenus dans une colonne de 4,2 cm de diamètre [éluant :acétate d'éthyle-cyclohexane (10-90 en volumes)] en utilisant une surpression de 40 kPa d'azote et en recueillant des fractions de 10 cm3. Les fractions 4 à 15 sont réunies et concentrées à sec sous pression réduite (2,7 kPa) à 40°C. On obtient ainsi 28,5 g de bromo-2 (chloro-2 phényl)-2 acétate de tert-butyle-(RS) à l'état d'huile utilisée telle quelle dans les synthèses ultérieures.

Le chlorure de bromo-2 (chloro-2 phényl)-2 acétyle-(RS) peut être préparé selon la méthode décrite à l'exemple 9, pour la préparation du chlorure de bromo-2 (chloro-4 phényl)-2 acétyle-(RS), mais à partir de 25 g d'acide (chloro-2 phényl)-2 acétique, de 7,8 cm3 de chlorure de thionyle et de 9,3 cm3 de brome. On obtient ainsi 39 g de chlorure de bromo-2 (chloro-2 phényl)-2 acétyle-(RS) utilisé tel quel pour l'étape suivante.

### Exemple 15

On opère comme à l'exemple 1, mais à partir de 1,04 g d'acide [(méthyl-3 phényl)-3 uréido]-2 acétique, de 1,8 g d' anilino-2 (bromo-3 phényl)-2 acétate de tert-butyle-(RS) et de 0,36 cm3 de chlorure de thionyle Le produit obtenu est purifié par chromatographie sur 100 g de silice (0,04-0,063 mm) contenus dans une colonne de 3 cm de diamètre [éluant : méthanol-dichloromé- thane (1-99 en volumes)] en utilisant une surpression de 40 kPa d'azote et en recueillant des fractions de 10 cm3. Les fractions 11 à 17 sont réunies et concentrées à sec sous pression réduite (2,7 kPa) à 40°C et le résidu obtenu est recristallisé dans le cyclohexane . On obtient ainsi 1,4 g de (bromo-3 phényl)-2 {[(méthyl-3 phényl)-3 uréido]-2 N-phényl-acétamido}-2 acétate de tert-butyle-(RS) fondant à 103°C.

L'anilino-2 (bromo-3 phényl)-2 acétate de tert-butyle-(RS) peut être préparé d'une manière analogue à celle décrite à l'exemple 8, pour la préparation de l'anilino-2 (méthoxy-4 phényl)-2 acétate de tert-butyle-(RS), mais à partir de 6 g de bromo-2 (bromo-3 phényl)-2 acétate de tert-butyle-(RS) et de 3,2 cm3 d'aniline dans 50 cm3 d'acétonitrile. On obtient ainsi 1,8 g d'anilino-2 (bromo-3 phényl)-2 acétate de tert-butyle-(RS) fondant à 90°C.

Le bromo-2 (bromo-3 phényl)-2 acétate de tert-butyle-(RS) peut être préparé d'une manière analogue à celle décrite à l'exemple 9, pour la préparation du bromo-2 (chloro-4 phényl)-2 acétate de tert-butyle-(RS), mais à partir de 7 g de chlorure de (bromo-3 phényl)-2 bromacétyle-(RS), de 9,5 cm3 de tertiobutanol et de 7,1 cm3 de N,N-diméthylaniline. Le produit obtenu est purifié par chromatographie sur 100 g de silice (0,04-0,063 mm) contenus dans une colonne de 3 cm de diamètre [éluant : acétate d'éthyle-cyclohexane (10-90 en volumes)] en utilisant une surpression de 40 kPa d'azote et en recueillant des fractions de 10 cm3. Les fractions 5 à 16 sont réunies et concentrées à sec sous pression réduite (2,7 kPa) à 40°C. On obtient ainsi 6 g de bromo-2 (bromo-3 phényl)-2 acétate de tert-butyle-(RS) à l'état d'huile utilisée telle quelle dans les synthèses ultérieures.

Le chlorure de bromo-2 (bromo-3 phényl)-2 acétyle-(RS) peut être préparé selon la méthode décrite à l'exemple 9, pour la préparation du chlorure de bromo-2 (chloro-4 phényl)-2 acétyle-(RS), mais à partir de 5 g d'acide (bromo-3 phényl)-2 acétique, de 2,8 cm3 de chlorure de thionyle et de 1,5 cm3 de brome. On obtient ainsi 7 g de chlorure de bromo-2 (chloro-3 phényl)-2 acétyle-(RS) utilisé tel quel pour l'étape suivante.

### Exemple 16

On opère comme à l'exemple 1, mais à partir de 2,1 g d'acide [(méthyl-3 phényl)-3 uréido]-2 acétique, de 3,02 g d' anilino-2 (fluoro-3 phényl)-2 acétate de tert-butyle-(RS) et de 0,72 cm3 de chlorure de thionyle. Le produit obtenu est purifié par chromatographie sur 100 g silice (0,04-0,063 mm) contenus dans une colonne de 3 cm de diamètre [éluant: méthanol-dichloromé- thane (1-99 en volumes)] en utilisant une surpression de 40 kPa d'azote et en recueillant des fractions de 10 cm3. Les fractions 10 à 16 sont réunies, concentrées à sec sous pression réduite (2,7 kPa) à 40°C et le résidu obtenu est recristallisé dans le cyclohexane. On obtient ainsi 1,3 g de (fluoro-3 phényl)-2 {[(méthyl-3 phényl)-3 uréido]-2 N-phényl-acétamido}-2 acétate de tert-butyle-(RS) fondant à 107°C.

L'anilino-2 (fluoro-3 phényl)-2 acétate de tert-butyle-(RS) peut être préparé d'une manière analogue à celle décrite à l'exemple 8, pour la préparation de l'anilino-2 (méthoxy-4 phényl)-2 acétate de tert-butyle-(RS),mais à partir de 11 g de bromo-2 (fluoro-3 phényl)-2 acétate de tert-butyle-(RS) et de 7 cm3 d'aniline dans 100 cm3 d'acétonitrile. On obtient ainsi 5,7 g d'anilino-2 (fluoro-3 phényl)-2 acétate de tert-butyle-(RS) fondant à 90°C.

Le bromo-2 (fluoro-3 phényl)-2 acétate de tert-butyle-(RS) peut être préparé d'une manière analogue à celle décrite à l'exemple 9, pour la préparation du bromo-2 (chloro-4 phényl)-2 acétate de tert-butyle-(RS), mais à partir de 15,5 g de chlorure de (fluoro-3 phényl)-2 bromacétyle-(RS), de 26,1 cm3 de tertiobutanol et de 19,4 cm3 de N,N-diméthylaniline. Le produit obtenu est purifié par chromatographie sur 250 g de silice (0,04-0,063 mm) contenus dans une colonne de 4,2 cm de diamètre [éluant : acétate d'éthyle-cyclohexane (10-90 en volumes)] en utilisant une surpression de 40 kPa d'azote et en recueillant des fractions de 10 cm3. Les fractions 7 à 14 sont réunies et concentrées à sec sous pression réduite (2,7 kPa) à 40°C. On obtient ainsi 11 g de bromo-2 (fluoro-3 phényl)-2 acétate de tert-butyle-(RS) à l'état d'huile utilisée telle quelle dans les synthèses ultérieures.

### Exemple 17

On opère comme à l'exemple 1, mais à partir de 11,6 g d'acide [(méthyl-3 phényl)-3 uréido]-2 acétique, de 15 g d' anilino-3 phényl-3 propionate d'éthyle-(RS) et de 4 cm3 de chlorure de thionyle. Le produit est purifié par chromatographie sur 250 g de silice (0,04-0,063 mm) contenus dans une colonne de 5 cm de diamètre [éluant: méthanol-dichlorométhane (2-98 en volumes)] en utilisant une surpression de 40 kPa d'azote et en recueillant des fractions de 75 cm3. Les fractions 19 à 21 sont réunies, concentrées à sec sous pression réduite (2,7 kPa) à 40°C et le résidu obtenu est recristallisé dans l'acétate d'éthyle. On obtient ainsi 4,5 g de {[(méthyl-3 phényl)-3 uréido]-2 N-phényl-acétamido}-3 phényl-3 propionate d'éthyle-(RS) fondant à 146°C.

L'anilino-3 phényl-3 propionate d'éthyle-(RS) peut être préparé de la façon suivante : à une solution de 22,3 g d'anilino-3 phényl-3 acrylate d'éthyle dans 400 cm3 de tétrahydrofuranne anhydre, maintenue à une température voisine de 0°C, on ajoute 12,4 cm3 d'acide trifluoroacétique puis on introduit en 20 minutes sous agitation, une solution de 10,4 g de cyanoborohydrure de sodium dans 60 cm3 de méthanol. Le mélange réactionnel est maintenu pendant 30 minutes à une température voisine de 0°C puis est versé sur 400 cm3 d'une solution aqueuse saturée de bicarbonate de sodium diluée par 500 cm3 d'eau. La phase aqueuse est extraite par 3 fois 150 cm3 d'acétate d'éthyle et les extraits organiques sont lavés par 30 fois 200 cm3 d'eau, séchés sur sulfate de magnésium et concentrés à sec sous pression réduite (2,7 kPa) à 45°C. On obtient ainsi 14,1 g d'anilino-3 phényl-3 propionate d'éthyle-(RS) fondant à 87°C.

L'anilino-3 phényl-3 acrylate d'éthyle peut être préparé selon la méthode décrite par F. TEXIER-BOULLET. , Synthesis, (1985), 679.

### Exemple 18

A une solution de 0,72 g d'amino-3 quinoléine dans 30 cm3 de dichlorométhane, on ajoute, à une température voisine de 0°C, 1 g de triéthylamine et 0,49 g de chloroformiate de trichlorométhyle. La suspension obtenue est agitée pendant 15 minutes à une température voisine de 0°C puis additionnée d'une solution de 1,3 g d'(amino-2 N-phényl-acétamido)-2 acétate de tert-butyle dans 10 cm3 de dichlorométhane. Le mélange réactionnel est agité pendant 3 heures à une température voisine de 20°C, puis additionnée de 20 cm3 d'eau. La phase aqueuse est séparée par décantation puis réextraite par 2 fois 15 cm3 de dichloro-1,2 éthane. Les phases organiques sont réunies, lavées par 2 fois 10 cm3 d'eau, séchées sur sulfate de magnésium , filtrées puis concentrées à sec sous pression réduite (2,7 kPa) à 40°C. L' huile obtenue est purifiée par chromatographie sur 50 g de silice (0,063-0,2 mm) contenus dans une colonne de 1,5 cm de diamètre [éluant: dichlorométhane-méthanol (98-2 en volumes)] en recueillant des fractions de 10 cm3. Les fractions ne contenant que le produit cherché sont réunies et concentrées à sec sous pression réduite (2,7 kPa) à 40°C. On obtient après recristallisation dans l'acétate d' éthyle, 0,8 g de {[(quinolyl-3)-3 uréido]-2 N-phényl-acétamido}-2 acétate de tert-butyle fondant à 191°C.

L'(amino-2 N-phényl-acétamido)-2 acétate de tert-butyle peut être préparé de la manière suivante : à une solution de 3,9 g de (N-phényl phtalimido-2 acétamido)-2 acétate de tert-butyle dans 70 cm3 de dichlorométhane, on ajoute, à une température voisine de 0°C, 1,4 g de méthylhydrazine. Le mélange réactionnel est agité pendant 48 heures à une température voisine de 20°C puis on ajoute 50 cm3 d'eau, agite, et sépare par décantation la phase aqueuse qui est réextraite par 2 fois 40 cm3 de dichlorométhane. Les phases organiques sont réunies, séchées sur sulfate de magnésium, filtrées puis concentrées à sec sous pression réduite (2,7 kPa) à 40°C. L'huile obtenue est purifiée par chromatographie sur 80 g de silice (0,063-0,2 mm) contenus dans une colonne de 2 cm de diamètre [éluant : acétate d'éthyle-méthanol (90-10 en volumes)] en recueillant des fractions de 20 cm3. Les fractions ne contenant que le produit cherché sont réunies et concentrées à sec sous pression réduite (2,7 kPa) à 40°C. On obtient ainsi 3,8 g d'(amino-2 N-phényl-acétamido)-2 acétate de tert-butyle sous forme d'une huile utilisée telle quelle dans les synthèses ultérieures.

Le (N-phényl phtalimido-2 acétamido)-2 acétate de tert-butyle peut être préparé de la manière suivante : à une solution de 94,9 g d'anilino-2 acétate de tert-butyle dans 360 cm3 de dichloro-1,2 éthane, maintenue sous atmosphère d'argon, on ajoute 42 g d' hydrogénocarbonate de sodium puis goutte à goutte, à une température voisine de 20°C, une solution de 102,4 g de chlorure de phtalimido-2 acétyle dans 400 cm3 de dichloro-1,2 éthane. La solution obtenue est agitée pendant 4 heures à une température voisine de 60°C, puis additionnée de 600 cm3 d' eau. La phase aqueuse est séparée par décantation puis réextraite par 2 fois 300 cm3 de dichloro-1,2 éthane. Les phases organiques sont réunies, séchées sur sulfate de magnésium, filtrées puis concentrées à sec sous pression réduite (2,7 kPa) à 40°C. On obtient ainsi, après recristallisation dans l' oxyde de diisopropyle, 152 g de (N-phényl phtalimido-2 acétamido)-2 acétate de tert-butyle fondant à 120°C.

L'anilino-2 acétate de tert-butyle peut être préparé de la manière suivante: à une solution de 196 g d' aniline dans 2100 cm3 d' acétonitrile, on ajoute 204,7 g de bromoacétate de tert-butyle. La solution obtenue est agitée à reflux pendant 4 heures. Après refroidissement, le produit insoluble est séparé par filtration et lavé par 3 fois 150 cm3 d' acétonitrile. Les filtrats sont réunis et concentrés à sec sous pression réduite (2,7 kPa) à 40°C. L'huile résiduelle est purifiée par distillation sous pression réduite (0,27 kPa). On obtient ainsi 133 g d' anilino-2 acétate de tert-butyle sous forme d' un liquide incolore qui distille à 122°C sous une pression de 0,27 kPa.

Le chlorure de phtalimido-2 acétyle peut être préparé selon la méthode décrite par W. GRASSMANN et coll., Der., 83, 244 (1950).

### Exemple 19

A une solution de 0,87 g d'acide quinoléinecarboxylique-2 dans 15 cm3 de dichlorométhane anhydre, on ajoute goutte à goutte, à une température voisine de 20°C, une solution de 0,85 g de N,N'-carbonyldiimidazole dans 10 cm3 de dichlorométhane anhydre. La suspension obtenue est agitée pendant 3 heures à une température voisine de 20°C puis on ajoute une solution de 2,6 g d'(amino-2 N-phényl-acétamido)-2 acétate de tert-butyle dans 25 cm3 de dichlorométhane anhydre. Le mélange réactionnel est agité pendant 16 heures à une température voisine de 20°C, puis additionné de 50 cm3 d'eau. La phase aqueuse est séparée par décantation puis réextraite par 2 fois 15 cm3 de dichloro-1,2 éthane. Les phases organiques sont réunies, lavées par 2 fois 25 cm3 d' une solution aqueuse saturée d'hydrogénocarbonate de sodium, puis par 2 fois 25 cm3 d'eau, séchées sur sulfate de magnésium, filtrées et concentrées à sec sous pression réduite (2,7 kPa) à 40°C. On obtient, après recristallisation du solide résiduel dans un mélange de cyclohexane et d'acétate d'éthyle (90-10 en volumes), 0,8 g de (N-phényl quinoléinecarboxamido-2 acétamido)-2 acétate de tert-butyle fondant à 114°C.

### Exemple 20

A une suspension de 0,51 g d'acide {[(méthyl-3 phényl)-3 uréido]-2 N-phényl-acétamido}-2 acétique et de 0,26 g d'hexafluoro-1,1,1,3,3,3 propanol-2 dans 30 cm³ de dichlorométhane, on ajoute 0,3 g de triéthylamine et 0,66 g d'hexafluorophosphonate de benzotriazolyl-1 oxy-tris-(diméthylamino) phosphonium. La solution est agitée pendant 2 heures à une température voisine de 25°C puis versée dans 50 cm³ d'une solution aqueuse saturée de chlorure de sodium. La phase organique est séparée et la phase aqueuse est extraite avec 3 fois 80 cm³ d'acétate d'éthyle. Les phases organiques réunies sont lavées successivement par 2 fois 30 cm3 d'une solution aqueuse d'acide chlorhydrique 2N, 2 fois 30 cm3 d'une solution aqueuse saturée d'hydrogénocarbonate de sodium, 50 cm3 d'une solution aqueuse saturée de chlorure de sodium, séchées sur sulfate de magnésium et concentrées à sec sous pression réduite (2,7 kPa) à 40°C. Le produit obtenu est purifié par chromatographie sur 40 g de silice (0,063-0,200 mm) contenus dans une colonne de 1,5 cm de diamètre [éluant : cyclohexane-acétate d'éthyle (50-50 en volumes)] en recueillant des fractions de 20 cm3. Les fractions 3 à 6 sont réunies et concentrées à sec sous pression réduite (2,7 kPa) à 40°C. On obtient, après recristallisaton dans l'oxyde de diisopropyle, 0,6 g de {[(méthyl-3 phényl)-3 uréido]-2 N-phényl-acétamido}-2 acétate d'hexafluoro-1,1,1,3,3,3 propyl-2 fondant à 112°C.

L'acide {[(méthyl-3 phényl)-3 uréido]-2 N-phényl-acétamido}-2 acétique peut être préparé de la manière suivante : à une solution de 3,3 g de {[(méthyl-3 phényl)-3 uréido]-2 N-phényl-acétamidol-2 acétate de tert-butyle dans 25 cm3 de dichlorométhane, on ajoute 7 g d'acide trifluoroacétique. La solution obtenue est chauffée à reflux pendant 4 heures puis concentrée à sec sous pression réduite (2,7 kPa) à 40°C. On obtient après recristallisation dans l'éther diisopropylique 2 g d'acide {[(méthyl-3 phényl)-3 uréido]-2 N-phényl-acétamido}-2 acétique.

### Exemple 21

On opère comme à l'exemple 1, mais à partir de 0,85 g d'acide [(méthyl-3 phényl)-3 uréido]-2 acétique, de 1,25 g d'anilino-2 malonate de di-tert-butyle et de 0,3 cm3 de chlorure de thionyle. Le produit obtenu est purifié par chromatographie sur 50 g de silice (0,04-0,063 mm) contenus dans une colonne de 3 cm de diamètre [éluant : méthanol-dichlorométhane (1,5-98,5 en volumes)] en utilisant une surpression de 40 kPa d'azote et en recueillant des fractions de 25 cm3. Les fractions 6 à 15 sont réunies, concentrées à sec sous pression réduite (2,7 kPa) à 35°C et le résidu obtenu est cristallisé dans 10cm3 d'oxyde de diéthyle. On obtient ainsi 0,67 g de {[(méthyl-3 phényl)-3 uréido]-2 N-phényl-acétamido}-2 malonate de di-tert-butyle fondant à 175°C.

L'anilino-2 malonate de di-tert-butyle peut être préparé d'une manière analogue à celle décrite à l'exemple 8, pour la préparation de l'anilino-2 (méthoxy-4 phényl)-2 acétate de tert-butyle-(RS), mais à partir de 2 g de bromo-2 malonate de di-tert-butyle et de 1,24 cm3 d'aniline dans 15 cm3 d'acétonitrile. Le produit obtenu est purifié par chromatographie sur 50 g de silice (0,04-0,063 mm) contenus dans une colonne de 3 cm de diamètre (éluant: dichlorométhane) en utilisant une surpression de 40 kPa d'azote et en recueillant des fractions de 25 cm3. Les fractions 3 à 6 sont réunies, concentrées à sec sous pression réduite (2,7 kPa) à 95°C. On obtient ainsi 1,3 g d'anilino-2 malonate de di-tert-butyle fondant à 94°C.

Le bromo-2 malonate de di-tert-butyle peut être préparé de la façon suivante : à une solution de 2,24 cm3 de malonate de di-tert-butyle et de 1,18 g d'acétamide dans 45 cm3 de chloroforme chauffée à reflux, on ajoute goutte à goutte 0,76 cm3 de brome et le mélange est chauffé à reflux pendant encore 11 heures. Après refroidissement, l'insoluble est séparé par filtration et le filtrat est lavé par 2 fois 10 cm3 d'une solution aqueuse saturée d'hydrogénocarbonate de sodium, séché sur sulfate de magnésium puis concentré à sec sous pression réduite (2,7 kPa) à 35°C. On obtient ainsi 2,1 g de bromo-2 malonate de di-tert-butyle à l'état d'huile utilisée telle quelle dans les synthèses ultérieures.

La présente invention concerne également les médicaments constitués par au moins un composé de formule (I) à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemples des crèmes, lotions, collyres, collutoires, gouttes nasales ou aérosols.

En thérapeutique humaine, les composés selon l'invention sont particulièrement utiles pour le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, de la maladie de Parkinson, de la diskinésie tardive, du syndrome du colon irritable, de la pancréatite aigue, des ulcères et des désordres de la motilité intestinale de certaines tumeurs de l'oesophage inférieur, du colon et de l'intestin, comme potentialisateur de l'activité analgésique des médicaments analgésiques narcotiques et non narcotiques et comme régulateur de l'appétit.

Les doses dépendent de l'effet recherché, de la durée du traitement et de la voie d'administration utilisée ; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter. Les exemples suivants illustrent des compositions selon l'invention :

### Exemple A

On prépare, selon la technique habituelle, des gélules dosées à 50 mg de produit actif ayant la composition suivante :
- (chloro-2 phényl)-2 {[(méthyl-3 phényl)-3 uréido]-2 N-phényl-acétamido}-2 acétate de tert-butyle-(RS) 50 mg
- cellulose 18 mg
- lactose 55 mg
- silice colloïdale 1 mg
- carboxyméthylamidon sodique 10 mg
- talc 10 mg
- stéarate de magnésium 1 mg

### Exemple B

On prépare, selon la technique habituelle, des comprimés dosés à 50 mg de produit actif ayant la composition suivante :
- (bromo-3 phényl)-2 {[(méthyl-3 phényl)-3 uréido]-2 N-phényl-acétamido}-2 acétate de tert-butyle-(RS) 50 mg
- lactose 104 mg
- cellulose 40 mg
- polyvidone 10 mg
- carboxyméthylamidon sodique 22 mg
- talc 10 mg
- stéarate de magnésium 2 mg
- silice colloïdale 2 mg
- mélange d'hydroxyméthylcellulose, glycérine, oxyde de titane (72-3,5-24,5) q.s.p 1 comprimé pelliculé terminé à 245 mg

### Exemple C

On prépare une solution injectable contenant 10 mg de produit actif ayant la composition suivante :
- (amino-4 phényl)-2 {[(méthyl-3 phényl)-3 uréido]-2 N-phényl-acétamido}-2 acétate de tert-butyle-(RS) 50 mg
- acide benzoïque 80 mg
- alcool benzylique 0,06 cm3
- benzoate de sodium 80 mg
- éthanol à 95 % 0,4 cm3
- hydroxyde de sodium 24 mg
- propylène glycol 1,6 cm3
- eau q.s.p 4 cm3

## Revendications (Revendications pour l'(les) Etat(s) contractant(s) suivant(s): AT, BE, CH, DE, DK, FR, GB, IT, LI, LU, NL, SE)

1. Composés de formule : 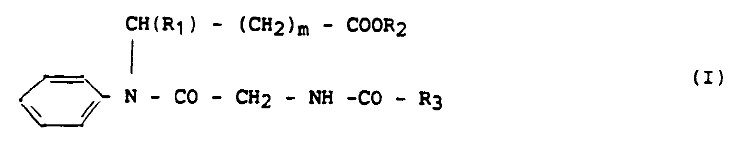 dans laquelle
- R₁ représente un atome d'hydrogène ou un radical alkyle, alcoxycarbonyle ou phényle non substitué ou substitué par un ou plusieurs subtituants choisis parmi les atomes d'halogène et les radicaux alkyle, alcoxy, nitro, amino et alkylthio,
- R₂ représente un radical alkyle (1-8C), polyfluoroalkyle, cinnamyle, ou cycloalkyle non substitué ou substitué par un ou plusieurs radicaux alkyle,
- R₃ représente un radical phényle (non substitué ou substitué par un ou plusieurs substituants choisis parmi les radicaux alkyle, alcoxy et alkylthio et les atomes d'halogène), un radical naphtyle, indolyle, quinolyle, phénylamino (dont le noyau phényle est éventuellement substitué par un ou plusieurs substituants choisis parmi les atomes d'halogène et les radicaux alkyle, alcoxy et alkylthio) ou un radical quinolylamino,
- m est égal à 0 ou 1, étant entendu que lorsque R₁ représente un atome d'hydrogène ou un radical alkyle ou phényle, R₃ représente un radical naphtyle, indolyle ou phénylamino éventuellement substitué par un radical alkyle, alcoxy, alkylthio ou par un ou deux atomes d'halogène et m est égal à 0, R₂ n'est pas un radical alkyle contenant 1 à 4 atomes de carbone ou un radical cycloalkyle ainsi que leurs racémiques et leurs énantiomères lorsqu'ils contiennent un ou plusieurs centres asymétriques.

2. Composés de formule (I) selon la revendication 1 pour lesquels:
- R₁ représente un atome d'hydrogène ou un radical alcoxycarbonyle ou phényle éventuellement substitué par un ou plusieurs substituants choisis parmi les atomes d'halogène et les radicaux alcoxy, nitro et amino,
- R₂ représente un radical alkyle, polyfluoroalkyle, cinnamyle, cycloalkyle non substitué ou substitué par un ou plusieurs radicaux alkyle,
- R₃ représente un radical phényle substitué par un radical alkyle ou par un atome d'halogène, un radical quinolyle
- m est égal à 0 ou 1
ainsi que leurs racémiques et leurs énantiomères lorsqu'ils contiennent un ou plusieurs centres asymétriques.

3. Procédé de préparation des composés de formule (I) selon la revendication 1 pour lesquels R₃ représente un radical phénylamino dont le noyau phényle est éventuellement substitué par un ou plusieurs substituants choisis parmi les atomes d'halogène et les radicaux alkyle, alcoxy et alkylthio caractérisé en ce que l'on fait réagir un acide de formule :
HOOC - CH₂ - NH - CO - R₃ (II)
dans laquelle R₃ a les mêmes significations que précédemment, sur une amine de formule : 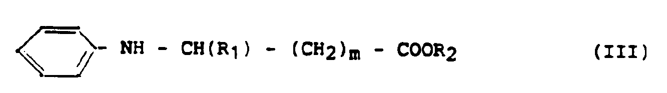 dans laquelle m, R₁ et R₂ ont les mêmes significations que dans la revendication 1, puis isole le produit.

4. Procédé de préparation des composés de formule (I) selon la revendication 1 pour lesquels R₃ représente un radical phénylamino dont le noyau phényle est éventuellement substitué par un ou plusieurs substituants choisis parmi les atomes d'halogène et les radicaux alkyle, alcoxy et alkylthio ou un radical quinolylamino caractérisé en ce que l'on fait réagir un dérivé aminé de formule : 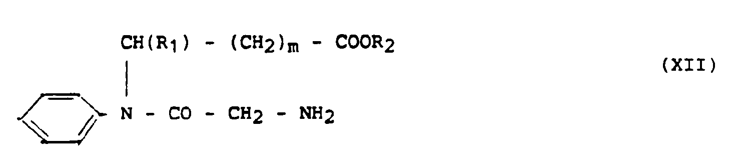 dans laquelle R₁, R₂ et m ont les mêmes significations que dans la revendication 1 sur un un dérivé de formule :
R₄ - NH -COOCCl₃ (XIII)
dans laquelle R₄ représente un radical phényle (éventuellement substitué par un ou plusieurs substituants choisis parmi les atomes d'halogène et les radicaux alkyle, alcoxy et alkylthio) ou quinolyle puis isole le produit.

5. Procédé de préparation des composés de formule (I) selon la revendication 1 pour lesquels R₃ représente un radical phényle (éventuellement substitué par un ou plusieurs substituants choisis parmi les radicaux alkyle, alcoxy et alkylthio et les atomes d'halogène), un radical naphtyle, indolyle ou quinolyle caractérisé en ce que l'on fait réagir un dérivé aminé de formule : 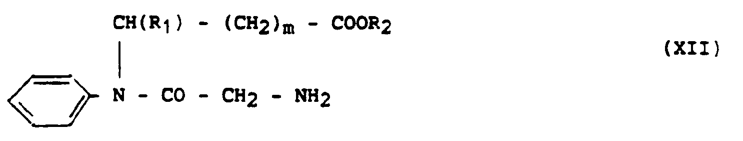 dans laquelle R₁, R₂ et m ont les mêmes significations que dans la revendication 1, sur un acide de formule :
HOOC - R₃ (XVII)
dans laquelle R₃ a les mêmes significations que précedemment ou un dérivé réactif de cet acide, puis isole le produit.

6. Procédé de préparation des composés de formule (I) selon la revendication 1, caractérisé en ce que l'on estérifie un acide de formule : 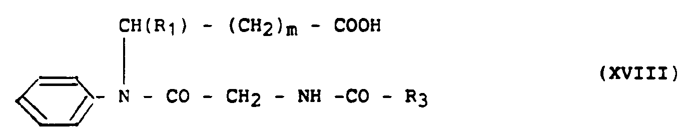 dans laquelle R₁, R₃ et m ont les mêmes significations que dans la revendication 1, puis isole le produit.

7. Procédé de préparation des composés de formule (I) selon la revendication 1 pour lesquels R₁ représente un radical phényle substitué par un radical amino caractérisé en ce que l'on réduit le dérivé nitré correspondant puis isole le produit.

8. Médicaments caractérisés en ce qu'ils contiennent en tant que principe actif au moins un composé de formule (I) selon la revendication 1.

9. Médicaments caractérisés en ce qu'ils contiennent en tant que principe actif au moins un composé de formule (I) selon la revendication 2.

10. Médicaments selon les revendications 8 et 9 pour le traitement et la prévention des désordres liés à la CCK et à la gastrine au niveau du système nerveux et de l'appareil gastrointestinal.

## Revendications (Revendications pour l'(les) Etat(s) contractant(s) suivant(s): ES, GR)

1. Procédé de préparation des composés de formule : 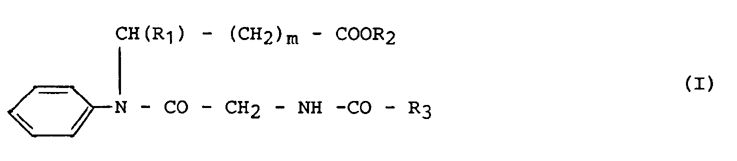 dans laquelle
- R₁ représente un atome d'hydrogène ou un radical alkyle, alcoxycarbonyle ou phényle non substitué ou substitué par un ou plusieurs subtituants choisis parmi les atomes d'halogène et les radicaux alkyle, alcoxy, nitro, amino et alkylthio,
- R₂ représente un radical alkyle (1-8C), polyfluoroalkyle, cinnamyle, ou cycloalkyle non substitué ou substitué par un ou plusieurs radicaux alkyle,
- R₃ représente un radical phényle (non substitué ou substitué par un ou plusieurs substituants choisis parmi les radicaux alkyle, alcoxy et alkylthio et les atomes d'halogène), un radical naphtyle, indolyle, quinolyle, phénylamino (dont le noyau phényle est éventuellement substitué par un ou plusieurs substituants choisis parmi les atomes d'halogène et les radicaux alkyle, alcoxy et alkylthio) ou un radical quinolylamino,
- m est égal à 0 ou 1,
étant entendu que lorsque R₁ représente un atome d'hydrogène ou un radical alkyle ou phényle, R₃ représente un radical naphtyle, indolyle ou phénylamino éventuellement substitué par un radical alkyle, alcoxy, alkylthio ou par un ou deux atomes d'halogène et m est égal à 0, R₂ n'est pas un radical alkyle contenant 1 à 4 atomes de carbone ou un radical cycloalkyle ainsi que leurs racémiques et leurs énantiomères lorsqu'ils contiennent un ou plusieurs centres asymétriques, caractérisé en ce que :
A - pour la préparation des composés de formule (I) dans laquelle R₃ représente un radical représente un radical phénylamino dont le noyau phényle est éventuellement substitué on fait réagir un acide de formule :
HOOC-CH₂-NH-CO-R₃ (II)
dans laquelle R₃ a les mêmes significations que précédemment, sur une amine de formule : 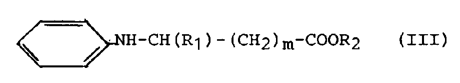 dans laquelle R₁, R₂ et m ont les mêmes significations que dans la formule (I) puis isole le produit,
B - pour la préparation des composés de formule (I) pour lesquels R₃ représente un radical phénylamino dont le noyau est éventuellement substitué par un ou plusieurs substituants choisis parmi les atomes d'halogène et les radicaux alkyle, alcoxy et alkylthio ou un radical quinolylamino, on fait réagir un dérivé aminé de formule : 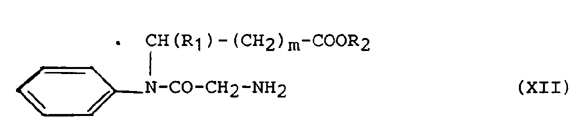 dans laquelle R₁, R₂ et m ont les mêmes significations que dans la formule (I), sur un dérivé de formule
R₄-NH-COOCCl₃ (XIII)
dans laquelle R₄ représente un radical phényle (éventuellement substitué par un ou plusieurs substituants choisis parmi les atomes d'halogène et les radicaux alkyle, alcoxy et alkylthio) ou quinolyle, puis isole le produit,
C - pour la préparation des composés de formule (I) pour lesquels R₃ représente un radical phényle éventuellement substitué par un ou plusieurs substituants choisis parmi les radicaux alkyle, alcoxy et alkylthio et les atomes d'halogène, un radical naphtyle, indolyle ou quinolyle on fait réagir un dérivé aminé de formule : 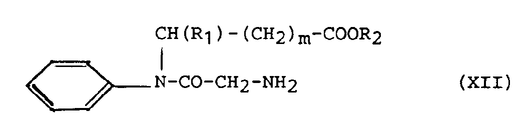 dans laquelle R_{1,} R₂ et m ont les mêmes significations que dans la formule (I), sur un acide de formule :
HOOC-R₃ (XVII)
dans laquelle R₃ a les mêmes significations que précédemment ou un dérivé réactif de cet acide, puis isole le produit,
D - pour la préparation des composés de formule (I) on estérifie un acide de formule : 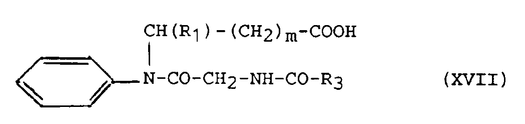 dans laquelle R_{1,} R₃ et m ont les mêmes signifcations que dans la formule (I), puis isole le produit,
E - pour la préparation des composés de formule (I) pour lesquels R₁ représente un radical phényle substitué par un radical amino, on réduit le dérivé nitré correspondant, puis isole le produit.

2. Procédé selon la revendication 1 pour la préparation des composés de formule (I) pour lesquels :
- R₁ représente un atome d'hydrogène ou un radical alcoxycarbonyle ou phényle éventuellement substitué par un ou plusieurs substituants choisis parmi les atomes d'halogène et les radicaux alcoxy, nitro et amino,
- R₂ représente un radical alkyle, polyfluoroalkyle, cinnamyle, cycloalkyle non substitué ou substitué par un ou plusieurs radicaux alkyle,
- R₃ représente un radical phényle substitué par un radical alkyle ou par un atome d'halogène, un radical quinolyle
- m est égal à 0 ou 1
ainsi que leurs racémiques et leurs énantiomères lorsqu'ils contiennent un ou plusieurs centres asymétriques.

## Patentansprüche (Patentansprüche für folgende(n) Vertragsstaat(en): AT, BE, CH, DE, DK, FR, GB, IT, LI, LU, NL, SE)

1. Verbindungen der Formel 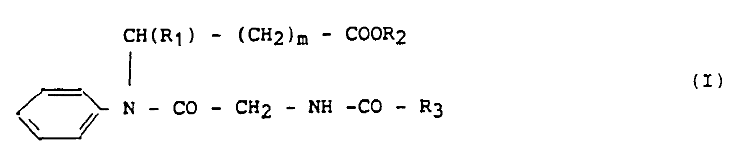 worin
- R₁ ein Wasserstoffatom oder einen Alkyl-, Alkoxycarbonyl- oder Phenylrest bedeutet, der unsubstituiert ist oder substituiert ist durch einen oder mehrere Substituenten, ausgewählt unter den Halogenatomen und den Alkyl-, Alkoxy-, Nitro-, Amino- und Alkylthioresten,
- R₂ einen Alkyl- (1-8 C), Polyfluoralkyl-, Cinnamyl- oder Cycloalkylrest, der unsubstituiert oder durch einen oder mehrere Alkylreste substituiert ist, bedeutet,
- R₃ einen Phenylrest (unsubstituiert oder substituiert durch einen oder mehrere Substituenten, ausgewählt unter den Alkyl-, Alkoxy- und Alkylthioresten und den Halogenatomen), einen Naphthyl-, Indolyl-, Chinolyl-, Phenylamino- (dessen Phenylkern gegebenenfalls durch einen oder mehrere Substituenten substituiert ist, ausgewählt unter den Halogenatomen und den Alkyl-, Alkoxy- und Alkylthioresten) oder einen Chinolylaminorest bedeutet,
- m 0 oder 1 ist,
mit der Maßgabe, daß, wenn R₁ ein Wasserstoffatom oder einen Alkyl- oder Phenylrest bedeutet, R₃ einen Naphthyl-, Indolyl- oder Phenylaminorest, welcher gegebenenfalls substituiert ist durch einen Alkyl-, Alkoxy-, Alkylthiorest oder durch ein oder zwei Halogenatome, bedeutet, und m für 0 steht, R₂ keinen Alkylrest mit 1 bis 4 Kohlenstoffatomen oder keinen Cycloalkylrest bedeutet, sowie deren Racemate und deren Enantiomere, wenn sie ein oder mehrere Asymmetriezentren enthalten.

2. Verbindungen der Formel (I) gemäß Anspruch 1 worin
- R₁ ein Wasserstoffatom oder einen Alkoxycarbonyl- oder Phenylrest, der gegebenenfalls substituiert ist durch einen oder mehrere Substituenten, ausgewählt unter den Halogenatomen und den Alkoxy-, Nitro- und Aminoresten, bedeutet,
- R₂ für einen Alkyl-, Polyfluoralkyl-, Cinnamyl-, Cycloalkylrest, der nicht substituiert oder durch einen oder mehrere Alkylreste substituiert ist, steht,
- R₃ einen Phenylrest, substituiert durch einen Alkylrest oder durch ein Halogenatom, einen Chinolylrest bedeutet,
- m für 0 oder 1 steht,
sowie deren Racemate und deren Enantiomere, wenn sie ein oder mehrere Asymmetriezentren enthalten.

3. Verfahren zur Herstellung der Verbindungen der Formel (I) gemäß Anspruch 1, worin R₃ einen Phenylaminorest, dessen Phenylkern gegebenenfalls durch einen oder mehrere Substituenten, ausgewählt unter den Halogenatomen und den Alkyl-, Alkoxy- und Alkylthioresten, substituiert ist, bedeutet, dadurch gekennzeichnet, daß man eine Säure der Formel
HOOC - CH₂ - NH - CO - R₃ (II)
worin R₃ die vorstehend angegebenen Bedeutungen besitzt, mit einem Amin der Formel 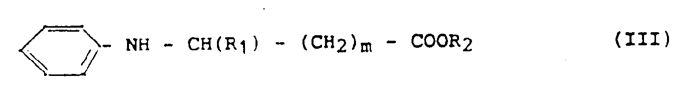 worin m, R₁ und R₂ die in Anspruch 1 angegebenen Bedeutungen besitzen, umsetzt, und anschließend das Produkt isoliert.

4. Verfahren zur Herstellung der Verbindungen der Formel (I) gemäß Anspruch 1, worin R₃ einen Phenylaminorest, dessen Phenylkern gegebenenfalls durch einen oder mehrere Substituenten, ausgewählt unter den Halogenatomen und den Alkyl-, Alkoxy- und Alkylthioresten, substituiert ist, oder einen Chinolylaminorest bedeutet, dadurch gekennzeichnet, daß man ein Aminoderivat der Formel 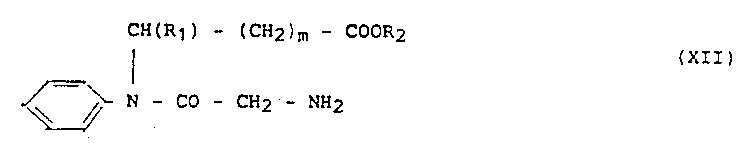 worin R₁, R₂ und m die in Anspruch 1 angegebenen Bedeutungen besitzen, mit einem Derivat der Formel
R₄ - NH -COOCCl₃ (XIII)
worin R₄ einen Phenylrest (gegebenenfalls substituiert durch einen oder mehrere Substituenten, ausgewählt unter den Halogenatomen und den Alkyl-, Alkoxy- und Alkylthioresten) oder Chinolylrest bedeutet, umsetzt, wonach man das Produkt isoliert.

5. Verfahren zur Herstellung der Verbindungen der Formel (I) gemäß Anspruch 1, worin R₃ einen Phenylrest (gegebenenfalls substituiert durch einen oder mehrere Substituenten, ausgewählt unter den Alkyl-, Alkoxy- und Alkylthioresten und den Halogenatomen), einen Naphthyl-, Indolyl- oder chinolylrest bedeutet, dadurch gekennzeichnet, daß man ein Aminoderivat der Formel 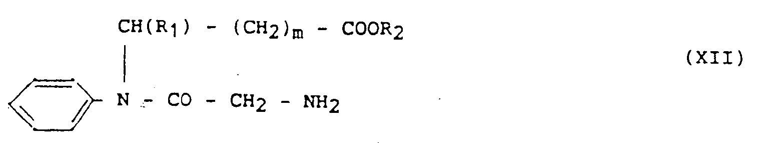 worin R₁, R₂ und m die in Anspruch 1 angegebenen Bedeutungen besitzen, mit einer Säure der Formel
HOOC - R₃ (XVII)
worin R₃ die vorstehend angegebenen Bedeutungen besitzt, oder einem reaktiven Derivat dieser Säure umsetzt, wonach man das Produkt isoliert.

6. Verfahren zur Herstellung der Verbindungen der Formel (I) gemäß Anspruch 1, dadurch gekennzeichnet, daß man eine Säure der Formel 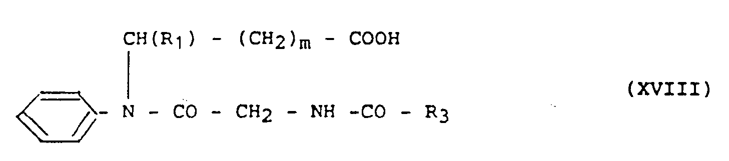 worin R₁, R₃ und m die in Anspruch 1 angegebenen Bedeutungen besitzen, verestert, wonach man das Produkt isoliert.

7. Verfahren zur Herstellung der Verbindungen der Formel (I) gemäß Anspruch 1, worin R₁ einen durch einen Aminorest substituierten Phenylrest bedeutet, dadurch gekennzeichnet, daß man das entsprechende Nitroderivat reduziert und danach das Produkt isoliert.

8. Arzneimittel, dadurch gekennzeichnet, daß sie als Wirkstoff zumindest eine Verbindung der Formel (I) gemäß Anspruch 1 enthalten.

9. Arzneimittel dadurch gekennzeichnet, daß sie als Wirkstoff zumindest eine Verbindung der Formel (I) gemäß Anspruch 2 enthalten.

10. Arzneimittel gemäß Anspruch 8 und 9 für die Behandlung und die Prävention von Störungen, die an das CCK und an das Gastrin im Bereich des Nervensystems und des gastrointestinalen Trakts gebunden sind.

## Patentansprüche (Patentansprüche für folgende(n) Vertragsstaat(en): ES, GR)

1. Verfahren zur Herstellung der Verbindungen der Formel 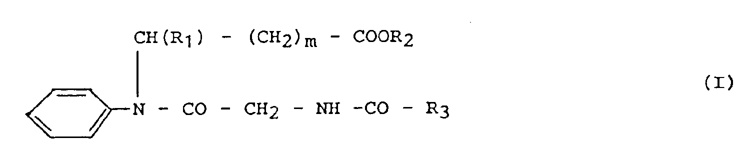 worin
- R₁ ein Wasserstoffatom oder einen Alkyl-, Alkoxycarbonyl- oder Phenylrest, der unsubstituiert ist oder substituiert ist durch einen oder mehrere Substituenten, ausgewählt unter den Halogenatomen und den Alkyl-, Alkoxy-, Nitro-, Amino- und Alkylthioresten, bedeutet,
- R₂ für einen Alkyl- (1-8 C), Polyfluoralkyl-, Cinnamyl- oder Cycloalkylrest, der unsubstituiert oder durch einen oder mehrere Alkylreste substituiert ist, steht,
- R₃ einen Phenylrest (unsubstituiert oder substituiert durch einen oder mehrere Substituenten, ausgewählt unter den Alkyl-, Alkoxy- und Alkylthioresten und den Halogenatomen), einen Naphthyl-, Indolyl-, Chinolyl-, Phenylaminorest (dessen Phenylkern gegebenenfalls durch einen oder mehrere Substituenten, ausgewählt unter den Halogenatomen und den Alkyl-, Alkoxy- und Alkylthioresten, substituiert ist) oder einen Chinolylaminorest bedeutet,
- m für 0 oder 1 steht,
mit der Maßgabe, daß, wenn R₁ ein Wasserstoffatom oder einen Alkyl- oder Phenylrest bedeutet, R₃ einen Naphthyl-, Indolyl- oder Phenylaminorest, der gegebenenfalls substituiert ist durch einen Alkyl-, Alkoxy-, Alkylthiorest oder durch ein oder zwei Halogenatome, bedeutet, und m für 0 steht, R₂ keinen Alkylrest mit 1 bis 4 Kohlenstoffatomen und keinen Cycloalkylrest bedeutet, sowie von deren Racematen und deren Enantiomeren, wenn sie einen oder mehrere Asymmetriezentren enthalten, dadurch gekennzeichnet, daß
A - zur Herstellung der Verbindung der Formel (I), worin R₃ einen Rest, dargestellt durch einen Phenylaminorest, bedeutet, dessen Phenylkern gegebenenfalls substituiert ist, man eine Säure der Formel
HOOC-CH₂-NH-CO-R₃ (II)
worin R₃ die vorstehend angegebenen Bedeutungen besitzt, mit einem Amin der Formel 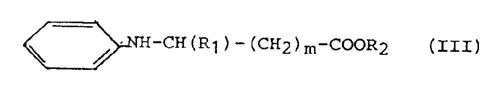 worin R₁, R₂ und m die für Formel (I) angegebenen Bedeutungen besitzen, umsetzt, wonach man das Produkt isoliert,
B - zur Herstellung der Verbindungen der Formel (I), worin R₃ einen Phenylaminorest, dessen Kern gegebenenfalls durch einen oder mehrere Substituenten, ausgewählt unter den Halogenatomen und den Alkyl-, Alkoxy- und Alkylthioresten, substituiert ist, oder einen Chinolylaminorest bedeutet, man ein Aminoderivat der Formel 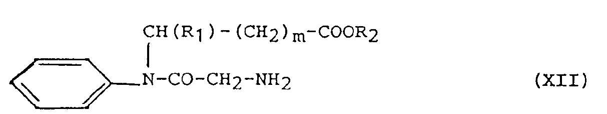 worin R₁, R₂ und m die für die Formel (I) angegebenen Bedeutungen besitzen, mit einem Derivat der Formel
R₄-NH-COOCCl₃ (XIII)
worin R₄ einen Phenylrest (gegebenenfalls substituiert durch einen oder mehrere Substituenten, ausgewählt unter den Halogenatomen und den Alkyl-, Alkoxy- und Alkylthioresten) oder einen Chinolylrest bedeutet, umsetzt, wonach man das Produkt isoliert,
C - zur Herstellung der Verbindungen der Formel (I), worin R₃ einen Phenylrest, gegebenenfalls substituiert durch einen oder mehrere Substituenten, ausgewählt unter den Alkyl-, Alkoxy- und Alkylthioresten und den Halogenatomen, einen Naphthyl-, Indolyl- oder Chinolylrest bedeutet, man ein Aminoderivat der Formel 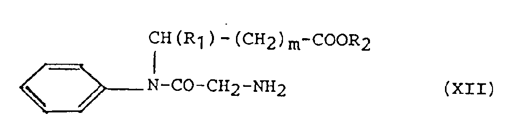 worin R₁, R₂ und m die für Formel (I) angegebenen Bedeutungen besitzen, mit einer Säure der Formel
HOOC -R₃ (XVII)
worin R₃ die vorstehend angegebenen Bedeutungen besitzt, oder einem reaktiven Derivat dieser Säure umsetzt, wonach man das Produkt isoliert,
D - zur Herstellung der Verbindungen der Formel (I) man eine Säure der Formel 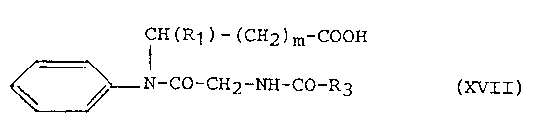 worin R₁, R₃ und m die für Formel (I) angegebenen Bedeutungen besitzen, verestert, wonach man das Produkt isoliert,
E - zur Herstellung der Verbindungen der Formel (I), worin R₁ einen durch eine Aminogruppe substituierten Phenylrest bedeutet, man das entsprechende Nitroderivat reduziert und danach das Produkt isoliert.

2. Verfahren gemäß Anspruch 1 zur Herstellung der Verbindungen der Formel (I), worin
- R₁ ein Wasserstoffatom oder einen Alkoxycarbonyl- oder Phenylrest, gegebenenfalls substituiert durch einen oder mehrere Substituenten, ausgewählt unter den Halogenatomen und den Alkoxy-, Nitro- und Aminoresten, bedeutet,
- R₂ für einen Alkyl-, Polyfluoralkyl-, Cinnamyl-, Cycloalkylrest, der unsubstituiert ist oder durch einen oder mehrere Alkylreste substituiert ist, steht,
- R₃ einen durch einen Alkylrest oder durch ein Halogenatom substituierten Phenylrest, einen Chinolylrest bedeutet,
- m für 0 oder 1 steht,
sowie von deren Racematen und deren Enantiomeren, wenn sie einen oder mehrere Asymmetriezentren enthalten.

## Claims (Claims for the following Contracting State(s): AT, BE, CH, DE, DK, FR, GB, IT, LI, LU, NL, SE)

1. Compounds of formula: 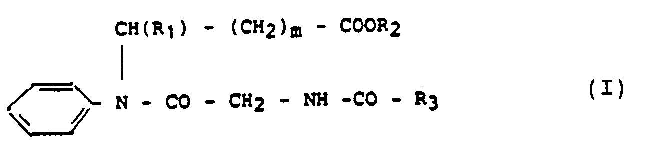 in which
- R₁ represents a hydrogen atom or an alkyl or alkoxycarbonyl radical or a phenyl radical which is unsubstituted or substituted by one or more substituents chosen from halogen atoms and alkyl, alkoxy, nitro, amino and alkylthio radicals,
- R₂ represents an alkyl (1-8 C), polyfluoroalkyl or cinnamyl radical or a cycloalkyl radical which is unsubstituted or substituted by one or more alkyl radicals,
- R₃ represents a phenyl radical (unsubstituted or substituted by one or more substituents chosen from alkyl, alkoxy and alkylthio radicals and halogen atoms), a naphthyl, indolyl, quinolyl or phenylamino radical (in which the phenyl ring is optionally substituted by one or more substituents chosen from halogen atoms and alkyl, alkoxy and alkylthio radicals) or a quinolylamino radical, and
- m is 0 or 1,
it being understood that when R₁ represents a hydrogen atom or an alkyl or phenyl radical, R₃ represents a naphthyl, indolyl or phenylamino radical, optionally substituted by an alkyl, alkoxy or alkylthio radical or by one or two halogen atoms, and m is 0, R₂ is not an alkyl radical containing 1 to 4 carbon atoms or a cycloalkyl radical, as well as their racemates and their enantiomers when they contain one or more centres of asymmetry.

2. Compounds of formula (I) according to claim 1, for which:
- R₁ represents a hydrogen atom or an alkoxycarbonyl radical or a phenyl radical optionally substituted by one or more substituents chosen from halogen atoms and alkoxy, nitro and amino radicals,
- R₂ represents an alkyl, polyfluoroalkyl or cinnamyl radical or a cycloalkyl radical which is unsubstituted or substituted by one or more alkyl radicals,
- R₃ represents a phenyl radical substituted by an alkyl radical or by a halogen atom, or a quinolyl radical, and
- m is 0 or 1
as well as their racemates and their enantiomers when they contain one or more centres of asymmetry.

3. Process for the preparation of compounds of formula (I) according to claim 1 for which R₃ represents a phenylamino radical in which the phenyl ring is optionally substituted by one or more substituents chosen from halogen atoms and alkyl, alkoxy and alkylthio radicals, characterised in that an acid of formula:
HOOC - CH₂ - NH - CO - R₃ (II)
in which R₃ has the same meanings as above, is reacted with an amine of formula: 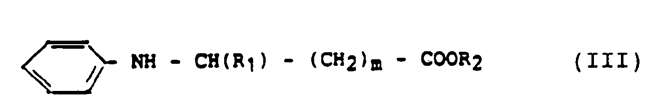 in which m, R₁ and R₂ have the same meanings as in claim 1, and the product is then isolated.

4. Process for the preparation of compounds of formula (I) according to claim 1 for which R₃ represents a phenylamino radical in which the phenyl ring is optionally substituted by one or more substituents chosen from halogen atoms and alkyl, alkoxy and alkylthio radicals, or a quinolylamino radical, characterised in that an amino derivative of formula: 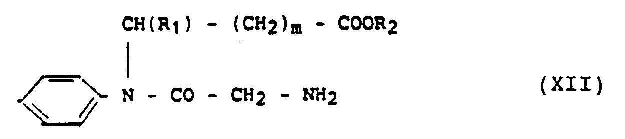 in which R₁, R₂ and m have the same meanings as in claim 1, is reacted with a derivative of formula:
R₄ - NH -COOCCl₃ (XIII)
in which R₄ represents a phenyl radical (optionally substituted by one or more substituents chosen from halogen atoms and alkyl, alkoxy and alkylthio radicals) or a quinolyl radical, and the product is then isolated.

5. Process for the preparation of compounds of formula (I) according to claim 1 for which R₃ represents a phenyl radical (optionally substituted by one or more substituents chosen from alkyl, alkoxy and alkylthio radicals and halogen atoms) or a naphthyl, indolyl or quinolyl radical, characterised in that an amino derivative of formula: 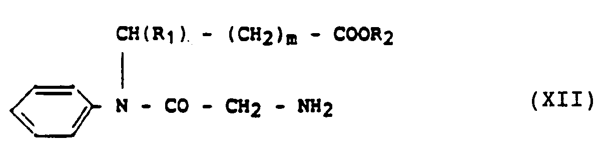 in which R₁, R₂ and m have the same meanings as in claim 1, is reacted with an acid of formula:
HOOC - R₃ (XVII)
in which R₃ has the same meanings as above, or a reactive derivative of this acid, and the product is then isolated.

6. Process for the preparation of compounds of formula (I) according to claim 1, characterised in that an acid of formula: 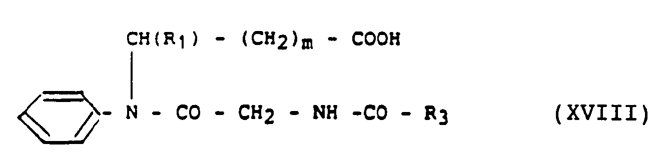 in which R₁, R₃ and m have the same meanings as in claim 1, is esterified and the product is then isolated.

7. Process for the preparation of compounds of formula (I) according to claim 1 for which R₁ represents a phenyl radical substituted by an amino radical, characterised in that the corresponding nitro derivative is reduced and the product is then isolated.

8. Medicaments, characterised in that they contain, as active principle, at least one compound of formula (I) according to claim 1.

9. Medicaments, characterised in that they contain, as active principle, at least one compound of formula (I) according to claim 2.

10. Medicaments according to claims 8 and 9 for the treatment and the prevention of disorders associated with CCK and with gastrin in the nervous system and the gastrointestinal system.

## Claims (Claims for the following Contracting State(s): ES, GR)

1. Process for the preparation of compounds of formula: 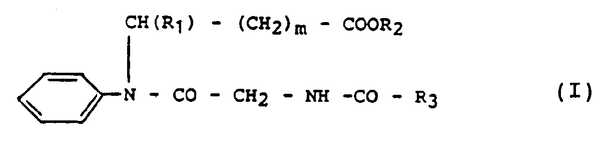 in which
- R₁ represents a hydrogen atom or an alkyl or alkoxycarbonyl radical or a phenyl radical which is unsubstituted or substituted by one or more substituents chosen from halogen atoms and alkyl, alkoxy, nitro, amino and alkylthio radicals,
- R₂ represents an alkyl (1-8 C), polyfluoroalkyl or cinnamyl radical or a cycloalkyl radical which is unsubstituted or substituted by one or more alkyl radicals,
- R₃ represents a phenyl radical (unsubstituted or substituted by one or more substituents chosen from alkyl, alkoxy and alkylthio radicals and halogen atoms), a naphthyl, indolyl, quinolyl or phenylamino radical (in which the phenyl ring is optionally substituted by one or more substituents chosen from halogen atoms and alkyl, alkoxy and alkylthio radicals) or a quinolylamino radical, and
- m is 0 or 1,
it being understood that when R₁ represents a hydrogen atom or an alkyl or phenyl radical, R₃ represents a naphthyl, indolyl or phenylamino radical, optionally substituted by an alkyl, alkoxy or alkylthio radical or by one or two halogen atoms, and m is 0, R₂ is not an alkyl radical containing 1 to 4 carbon atoms or a cycloalkyl radical, as well as their racemates and their enantiomers when they contain one or more centres of asymmetry, characterised in that:
A - for the preparation of compounds of formula (I) for which R₃ represents a phenylamino radical in which the phenyl ring is optionally substituted, an acid of formula:
HOOC - CH₂ - NH - CO - R₃ (II)
in which R₃ has the same meanings as above, is reacted with an amine of formula: 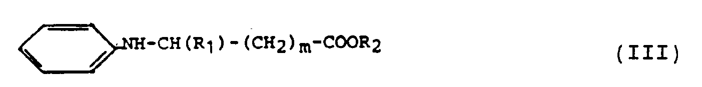 in which R₁, R₂ and m have the same meanings as in formula (I), and the product is then isolated,
B - for the preparation of compounds of formula (I) for which R₃ represents a phenylamino radical in which the ring is optionally substituted by one or more substituents chosen from halogen atoms and alkyl, alkoxy and alkylthio radicals, or a quinolylamino radical, an amino derivative of formula: 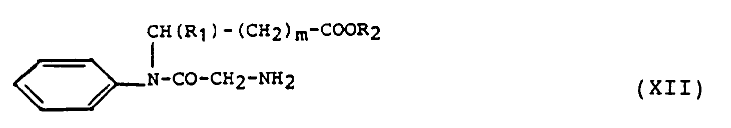 in which R₁, R₂ and m have the same meanings as in formula (I), is reacted with a derivative of formula:
R₄-NH-COOCCl₃ (XIII)
in which R₄ represents a phenyl radical (optionally substituted by one or more substituents chosen from halogen atoms and alkyl, alkoxy and alkylthio radicals) or a quinolyl radical, and the product is then isolated,
C - for the preparation of compounds of formula (I) for which R₃ represents a phenyl radical optionally substituted by one or more substituents chosen from alkyl, alkoxy and alkylthio radicals and halogen atoms or a naphthyl, indolyl or quinolyl radical, an amino derivative of formula: 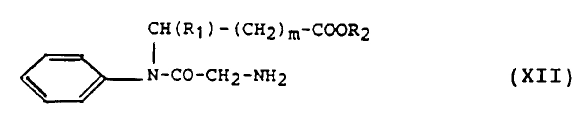 in which R₁, R₂ and m have the same meanings as in formula (I), is reacted with an acid of formula:
HOOC - R₃ (XVII)
in which R₃ has the same meanings as above, or a reactive derivative of this acid, and the product is then isolated,
D - for the preparation of compounds of formula (I), an acid of formula: 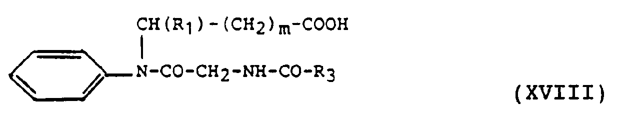 in which R₁, R₃ and m have the same meanings as in formula (I), is esterified, and the product is then isolated,
E - for the preparation of compounds of formula (I) for which R₁ represents a phenyl radical substituted by an amino radical, the corresponding nitro derivative is reduced, and the product is then isolated.

2. Process according to claim 1 for the preparation of compounds of formula (I), for which:
- R₁ represents a hydrogen atom or an alkoxycarbonyl radical or a phenyl radical optionally substituted by one or more substituents chosen from halogen atoms and alkoxy, nitro and amino radicals,
- R₂ represents an alkyl, polyfluoroalkyl or cinnamyl radical or a cycloalkyl radical which is unsubstituted or substituted by one or more alkyl radicals,
- R₃ represents a phenyl radical substituted by an alkyl radical or by a halogen atom, or a quinolyl radical, and
- m is 0 or 1
as well as their racemates and their enantiomers when they contain one or more centres of asymmetry.
